# EUROPÄISCHE PATENTSCHRIFT

(11) **EP 3 791 774 B1**
(45) Veröffentlichungstag und Bekanntmachung des Hinweises auf die Patenterteilung: **03.07.2024**
(21) Anmeldenummer: 20204649.6
(22) Anmeldetag: 02.12.2016
(51) Int. Cl.: A61B 1/00, G02B 23/24, G06F 3/01, G06F 3/0362, G06F 3/0338

(54) **BEOBACHTUNGSVORRICHTUNG, INSBESONDERE MEDIZINISCHE BEOBACHTUNGSVORRICHTUNG, MIT EINER BEDIENEINHEIT SOWIE VERWENDUNG EINES EINGABEMODULS**
OBSERVATION DEVICE, IN PARTICULAR MEDICAL OBSERVATION APPARATUS, WITH A CONTROL UNIT AND USE OF AN INPUT MODULE
DISPOSITIF D'OBSERVATION, EN PARTICULIER DISPOSITIF D'OBSERVATION MÉDICAL, DOTÉ D'UNE UNITÉ DE COMMANDE AINSI QU'UTILISATION D'UN MODULE D'ENTRÉE

(30) Priorität: 03.12.2015 DE 102015121017
(43) Veröffentlichungstag der Anmeldung: 17.03.2021
(62) Teilanmeldung aus: 16202058.0
(73) Patentinhaber: Karl Storz SE & Co. KG, 78532 Tuttlingen (DE)
(72) Erfinder: Köhler, Benedikt, 78532 Tuttlingen (DE); Heni, Andreas, 78532 Tuttlingen (DE); Kupferschmid, Markus, 78532 Tuttlingen (DE); Baum, Eckhart, 78532 Tuttlingen (DE); Schwarz, Peter, 78532 Tuttlingen (DE)
(74) Vertreter: Witte, Weller & Partner Patentanwälte mbB

(56) Entgegenhaltungen:
- DE-A1-102007 045 669
- DE-A1-102011 078 967
- US-A- 5 876 325
- US-A1- 2006 255 683
- US-A1- 2008 231 692
- US-A1- 2015 031 953

## Beschreibung

Die vorliegende Erfindung betrifft eine Beobachtungsvorrichtung, insbesondere eine medizinische Beobachtungsvorrichtung, die eine Bildaufnahmeeinheit, eine Wiedergabeeinheit, eine Bildverarbeitungseinheit sowie eine Bedieneinheit umfasst. Ferner betrifft die Erfindung eine Verwendung eines Mehrachs-Eingabemoduls.

Im Sinne der vorliegenden Erfindung können Beobachtungsvorrichtungen Endoskope, Exoskope sowie vergleichbare optische Instrumente umfassen. Vorzugsweise handelt es sich dabei um optische Instrumente, die mit Bildaufnehmern bzw. Bildsensoren ausgestattet sind, um ein digitales Abbild eines zu beobachtenden Objektes zu generieren. Derartige Instrumente sind regelmäßig als sog. okularlose Instrumente gestaltet. Mit anderen Worten umfassen diese Instrumente keinen klassischen rein optischen Strahlengang zwischen dem Objektiv und einem Auge eines Benutzers.

Stattdessen umfassen okularlose Instrumente regelmäßig Wiedergabeeinheiten in Form von Bildschirmen, Videobrillen (head-mounted displays) oder dgl. Dies kann verschiedene Vorteile mit sich bringen. Beispielhaft kann ohne weiteres eine Mehrzahl von Wiedergabeeinheiten angekoppelt werden, die das gleiche Bildsignal nutzen können.

Es sind auch okularlose Instrumente bekannt, die zur stereoskopischen Darstellung ausgebildet sind. Derartige Instrumente weisen beispielhaft zwei Bildaufnehmer auf, die einander benachbart angeordnet sind und zueinander einen definierten Versatz (Abstand oder Versatzwinkel) aufweisen.

Bildaufnehmer können generell mit einer geeigneten Eingangsstrahlenoptik gekoppelt sein. Auf diese Weise kann eine gewünschte optische Abbildung bewerkstelligt werden.

Die vorliegende Offenbarung bezieht sich insbesondere auf Beobachtungsvorrichtungen mit kompakten Bildaufnahmeeinheiten. Sowohl bei Endoskopen als auch bei Exoskopen wird auf geringe Abmessungen Wert gelegt, insbesondere am distalen Ende der Instrumente. Dies betrifft bei Endoskopen insbesondere einen Querschnitt oder Durchmesser des distalen Endes. Endoskope sind regelmäßig dazu ausgestaltet, in Körperöffnungen eingeführt zu werden. Um die Belastung für Patienten möglichst gering zu halten, werden geringe Abmessungen angestrebt. Das distale Ende eines Endoskops ist allgemein als anwenderfernes bzw. beobachterfernes Ende zu verstehen. Ein dem distalen Ende gegenüberliegendes proximales Ende ist allgemein als anwendernahes bzw. beobachternahes Ende zu verstehen.

Ein Exoskop kann allgemein auch als Mikroskop bezeichnet werden. Exoskope sind regelmäßig dazu ausgestaltet, ein Zielobjekt von außerhalb des Körpers aus einem definierten Arbeitsabstand, der etwa 25 bis 75 cm betragen kann, zu beobachten. Gleichwohl wird auch bei Exoskopen angestrebt, Bildaufnahmeeinheiten, Objektive bzw. allgemein das distale (beobachterferne) Ende des Instruments kompakt zu gestalten. Auf diese Weise wird etwa einem Operateur ein möglichst unverbautes Sichtfeld bereitgestellt. Auch wenn ein Exoskop in einem bestimmten Arbeitsabstand von einem Objekt (etwa einer zu beobachtenden Operationsstelle) angeordnet ist, so soll doch die Zugänglichkeit der Operationsstelle möglichst weiträumig gewährleistet sein.

Die gewünschte Kompaktheit führt dazu, dass bei okularlosen Endoskopen und Exoskopen häufig auf aufwendige Einrichtungen zur Veränderung der Brennweite (optischer Zoom) verzichtet wird. Es sind jedoch Anwendungsfälle denkbar, bei denen lediglich ein Ausschnitt eines aktuell bei gegebener Brennweite erfassten Objektfeldes von Interesse ist. Sowohl bei Endoskopen als auch bei Exoskopen kann der Arbeitsabstand jedoch häufig nicht beliebig variiert werden. Aufgrund ihrer Kompaktheit können optische Instrumente, die etwa als Endoskop oder Exoskop ausgestaltet sind, von Hand geführt werden. Gleichwohl sind diverse Anwendungsfälle denkbar, bei denen das Instrument fest an einem Stativ oder einer ähnlichen Haltevorrichtung aufgenommen ist. Dies ist insbesondere dann der Fall, wenn ein möglichst verwacklungsfreies Bild gewünscht ist. Ferner hat die Fixierung des Instruments den Vorteil, dass einem Operateur oder einer Hilfsperson beide Hände für andere Tätigkeiten zur Verfügung stehen.

Bei okularlosen Instrumenten, insbesondere bei okularlosen Exoskopen oder Endoskopen, besteht die Möglichkeit, Bildaufnehmer bzw. Bildsensoren sehr nahe am Objektiv der Instrumente anzuordnen. Da kein (optisches) Okular vorgesehen sein muss, ist es nicht erforderlich, einen optischen Strahlengang durch das Instrument bzw. durch einen beträchtlichen Teil des Instruments hindurch zu führen. Mit anderen Worten kann ein Abbild des zu beobachtenden Objekts nahe am distalen Ende des Instruments erfasst und in elektrische Signale überführt werden, auf deren Basis Bilddaten ableitbar sind. Dies führt regelmäßig dazu, dass auch am Instrument selbst gewisse Datenverarbeitungskapazitäten vorzusehen sind. Dies kann zumindest eine begrenzte Rechenkapazität umfassen. Ferner können etwa Pufferspeicher und ähnliche Baugruppen vorgesehen sein.

Ein okularloses Instrument weist üblicherweise ferner eine Schnittstelle auf, über die Bilddaten einer Wiedergabeeinheit oder einer (externen) Bildverarbeitungseinheit zuführbar sind. Bei den Bilddaten kann es sich um sog. Rohdaten handeln. Die Bilddaten können jedoch auch bereits im Instrument selbst bearbeitet bzw. verarbeitet werden.

Es ist kein klassisches Okular vorgesehen, über das ein Beobachtungsstrahlengang einem menschlichen Auge (etwa eines Operateurs) direkt sichtbar gemacht wird. Bei einem okularlosen Instrument im Sinne der vorliegenden Offenbarung erfolgt eine Wandlung bzw. Transformation von optischen Signalen des Beobachtungsfeldes in Bilddaten, die dann wiederum in optische Signale überführt werden, etwa zur Darstellung an einem Bildschirm.

Ein Exoskop im Sinne der vorliegenden Offenbarung ist beispielhaft aus der DE 10 2013 110 543 A1 bekannt. Das vorbekannte Exoskop ist als Stereo-Exoskop ausgebildet und umfasst eine Stereo-Optik zur Aufnahme eines Stereobildes.

Die DE 10 2011 078 967 A1 offenbart eine Mikroskopievorrichtung mit einem Operationsmikroskop mit Kamera, einem das Operationsmikroskop sowie die Kamera tragenden Stativ, und einer von der Kamera räumlich getrennten Bedieneinheit zur Steuerung von Gerätefunktionen der Kamera, wobei die Bedieneinheit einen Bildschirm zur Darstellung von aufrufbaren Gerätefunktionen und ein als Dreh-Drück-Bedienmittel ausgebildetes Auswahl- und Aufrufmittel zum Auswählen und Aufrufen einer der dargestellten Gerätefunktionen aufweist, wobei die Bedieneinheit so eingerichtet ist, dass durch Drehen des Dreh-Drück-Bedienmittels eine der dargestellten Gerätefunktionen ausgewählt wird und durch Drücken des Dreh-Drück-Bedienmittels die ausgewählte Gerätefunktion aufgerufen wird. Die DE 10 2011 078 967 A1 lehrt grundsätzlich, die Bedieneinheit zur Steuerung weiterer Funktionen der Mikroskopievorrichtung neben der Kamera zu nutzen, beispielsweise zur Steuerung von Bewegungsfunktionen des Operationsmikroskops. Jedoch lehrt die DE 10 2011 078 967 A1 keine direkte Steuerung von Bewegungsachsen über das Dreh-Drück-Bedienmittel, sondern allenfalls eine Auswahl konkret anzusteuernder Achsen sowie entsprechende Bewegungsvorgaben für diese Achsen mit dem Dreh-Drück-Bedienmittel. Damit wird zwar eine Einhand-Bedienung ermöglicht. Es ist jedoch nicht möglich, die Mikroskopievorrichtung intuitiv und unmittelbar zu verfahren.
Aus der US 2006/0255683 A1 ist ein Controller mit haptischer Rückmeldung zur Steuerung eines Geräts bekannt, der eine Basis, eine in Bezug auf die Basis rotierbare Kappe, einen piezoelektrischen Motor mit einem an der Basis befestigten Stator und einem an der Kappe befestigten Rotor, und eine Steuerungsvorrichtung aufweist, wobei die Steuerungsvorrichtung dazu ausgebildet ist, über den piezoelektrischen Motor eine haptische Rückmeldung auszugeben.

Vor diesem Hintergrund liegt der Erfindung die Aufgabe zugrunde, eine Beobachtungsvorrichtung, insbesondere eine medizinische Beobachtungsvorrichtung, anzugeben, die einfach und fehlerarm bedienbar ist und eine kompakte Bauform zumindest einer Bildaufnahmeeinheit der Beobachtungsvorrichtung erlaubt. Vorzugsweise soll die Beobachtungsvorrichtung gleichwohl eine erweiterte Funktionalität aufweisen, insbesondere die Möglichkeit zur Wahl und Variation einer Vergrößerung der Abbildung des Objektfeldes. Darüber hinaus ist die Bedieneinheit vorzugsweise derart funktional mit der Beobachtungsvorrichtung gekoppelt, dass eine intuitive Bedienung zumindest von Teilfunktionen betreffend die Aufnahme und von Teilfunktionen betreffend die Wiedergabe von Bildern ermöglicht ist. Mit anderen Worten ergibt sich vorzugsweise eine Bedienlogik, bei der ein Bediener, ohne dies bewusst zu merken, in einfacher Weise auf durch die Bildaufnahmeeinheit bereitgestellte Aufnahmebilder sowie auf durch die Wiedergabeeinheit bereitgestellte Wiedergabebilder einwirken kann. Hierbei ist es vorzugsweise aus Sicht des Bedieners zunächst einmal unerheblich, ob er beispielsweise direkt auf eine Optik der Bildaufnahmeeinheit einwirkt oder über seine Betätigung der Bedieneinheit die Signalverarbeitung bzw. Datenverarbeitung, die aufgenommenen Bilder betreffend, beeinflusst.

Vorzugsweise erlaubt die Bedieneinheit eine Kopplung der Bildaufnahmeeinheit mit der Bildverarbeitungseinheit und der Wiedergabeeinheit, zumindest im Hinblick auf die Bedienung, die die Teilsysteme funktional miteinander verbindet und sozusagen eine einheitliche/integrale Steuerung des gekoppelten Systems ermöglicht.

Ferner liegt der Erfindung die Aufgabe zugrunde, vorteilhafte Verwendungen eines Eingabemoduls bei einer Bedieneinheit für eine Beobachtungsvorrichtung anzugeben, wobei das Eingabemodul, aus Bedienersicht, vorzugsweise eine Kopplung von Teilsystemen der Beobachtungsvorrichtung erlaubt, so dass der Bediener über lediglich ein Eingabemodul integral auf die Bildaufnahmeeinheit, die Wiedergabeeinheit und die Bildverarbeitungseinheit der Beobachtungsvorrichtung einwirken kann.
Die Aufgabe der Erfindung wird eine Beobachtungsvorrichtung gemäß dem unabhängigen Vorrichtungsanspruch und durch eine Verwendung eines Eingabemoduls gemäß dem unabhängigen Verwendungsanspruch gelöst.

Die vorliegende Erfindung bezieht sich allgemein auf eine Beobachtungsvorrichtung, insbesondere eine medizinische Beobachtungsvorrichtung, die Folgendes aufweist:
- eine Bildaufnahmeeinheit mit zumindest einem Bildaufnehmer, vorzugsweise einer Stereo-Bildaufnahmeeinheit mit zwei Bildaufnehmern,
- eine Wiedergabeeinheit, die zur Wiedergabe von Bilddaten ausgestaltet ist, die durch die Bildaufnahmeeinheit bereitgestellt werden,
- eine Bildverarbeitungseinheit für Bildverarbeitungsprozesse,
- eine Bedieneinheit mit einem Mehrachs-Eingabemodul, und
- einen Positionierantrieb für die Bildaufnahmeeinheit,

wobei Bildaufnahmeparameter und Wiedergabeparameter über das Eingabemodul steuerbar sind,
wobei das Eingabemodul zumindest in einem ersten Betriebsmodus und einem zweiten Betriebsmodus betreibbar ist,
wobei im ersten Betriebsmodus eine direkte Steuerung von Bildaufnahmeparametern und Wiedergabeparametern ermöglicht ist,
wobei das Eingabemodul im zweiten Betriebsmodus betätigbar ist, um den Positionierantrieb zur Positionierung der Bildaufnahmeeinheit zu steuern,
wobei das Eingabemodul als Einhand-Eingabemodul ausgestaltet ist und ein durch einen Bediener manipulierbares Betätigungselement aufweist, das mehrere Bewegungsfreiheitsgrade für Eingaben bereitstellt, und
wobei verschiedene Achsen des Positionierantriebs gleichzeitig durch das Eingabemodul steuerbar sind.

Erfindungsgemäß erlaubt nämlich das Mehrachs-Eingabemodul der Bedieneinheit eine einfache, integrale Bedienung der Beobachtungsvorrichtung. Hierbei ist es zunächst unerheblich, ob ein Bediener (etwa ein Operateur oder eine Hilfsperson) durch eine aktuelle Eingabe auf die Bildaufnahmeeinheit, die Bildverarbeitungseinheit oder die Wiedergabeeinheit einwirkt. Da das Eingabemodul als Mehrachs-Eingabemodul ausgestaltet ist, kann gleichzeitig oder nahezu gleichzeitig auf die Bildaufnahmeeinheit, die Wiedergabeeinheit und/oder die Bildverarbeitungseinheit eingewirkt werden. Das Ergebnis einer solchen Eingabe wird für den Bediener unmittelbar durch eine Veränderung des wiedergegebenen Bildes sichtbar. Hierbei ist es unerheblich, ob die Veränderung des wiedergegebenen Bildes durch eine Einflussnahme auf die Bildaufnahmeeinheit, die Bildverarbeitungseinheit oder die Wiedergabeeinheit ausgelöst wurde. Die Kopplung zwischen dem Eingabemodul und der Bildaufnahmeeinheit kann mittelbar oder unmittelbar erfolgen. Die Kopplung zwischen dem Eingabemodul und der Bildaufnahmeeinheit ist funktioneller oder struktureller Art.

Bei dem Wiedergabeparameter kann es sich beispielhaft um eine aktuell gewählte Vergrößerung (Zoom-Faktor) handeln. Der Wiedergabeparameter betrifft eine aktuelle Position eines wiedergegebenen Bildes in einem bereitgestellten globalen Aufnahmebild. Um derartige Wiedergabeparameter zu steuern, kann das Eingabemodul mittelbar oder unmittelbar auf die Bildverarbeitungseinheit und/oder die Wiedergabeeinheit einwirken. Der Bildaufnahmeparameter kann etwa eine aktuelle Lage bzw. einen aktuellen Abstand einer Schärfeebene oder allgemein einen Fokusparameter betreffen. Beispielhaft ist die Bildaufnahmeeinheit mit einem Fokusantrieb versehen. Demgemäß kann das Eingabemodul mittelbar oder unmittelbar mit der Bildaufnahmeeinheit gekoppelt werden, um den Fokusantrieb zu steuern. Der Bildaufnahmeparameter kann ferner jedoch auch eine Beleuchtung des aktuell beobachteten Objektfeldes betreffen. Ferner kann der Bildaufnahmeparameter etwa eine Aktivierung von Filtern, Blenden, Spiegeln oder Ähnliches betreffen.

In zumindest einigen beispielhaften Ausgestaltungen ist der zumindest eine Sensor zur Erfassung einfallender elektromagnetischer Strahlung in zumindest dem UV-Bereich, dem Bereich sichtbaren Lichts, und/oder dem (N)IR-Bereich ausgebildet. Demgemäß bezieht sich die vorliegende Offenbarung aus die Bilderfassung durch die Erfassung elektromagnetischer Strahlung, die im Wesentlichen durch ein zu beobachtendes Objekt reflektiert wurde, zum Beispiel durch ein Körperteil oder ein Organ eines Patienten.

Der zumindest eine Sensor kann dazu ausgestaltet sein, Licht in zumindest einem Abschnitt des Wellenlängenbereichs zwischen etwa 10 nm (Nanometer) und etwa 1400 nm zu erfassen. Ultraviolettes Licht (UV) umfasst elektromagnetische Strahlung im Bereich von etwa 10 nm bis etwa 400 nm. Sichtbares Licht umfasst elektromagnetische Strahlung im Bereich von etwa 400 nm bis etwa 700 nm. Licht im Nahinfrarotbereich (NIR) umfasst elektromagnetische Strahlung im Bereich von etwa 700 nm bis etwa 1400 nm.

In einer beispielhaften Ausgestaltung, die Gegenstand eines bevorzugten Ausführungsbeispiels ist, ist das Eingabemodul ferner zur Steuerung zumindest eines Wiedergabeparameters mit der Bildaufnahmeeinheit koppelbar. Gemäß dieser Ausgestaltung erfolgt also die Bildverarbeitung bereits zumindest teilweise beim optischen Instrument selbst bzw. bei dessen Bildaufnahmeeinheit. Der Wiedergabeparameter betrifft beispielsweise einen Abbildungsmaßstab (Vergrößerungsfaktor bzw. Zoom-Faktor). Sofern ein gewünschter Abbildungsmaßstab oder Bildausschnitt durch gezielte Auswahl definierter Mengen oder Teilmengen der Aufnahmepixelmenge erzeugbar ist, kann auch die Bildaufnahmeeinheit selbst zur Bildverarbeitung herangezogen werden, um gewünschten Wiedergabeparametern zu entsprechen. In ähnlicher Weise wird eine Bildposition verändert, wenn ein Ausschnitt der Aufnahmepixelmenge innerhalb des durch die Aufnahmepixelmenge bereitgestellten Bereichs verschoben bzw. neu positioniert wird.

Mit anderen Worten ist gemäß der Erfindung das optische Instrument selbst bzw. dessen Bildaufnahmeeinheit zumindest teilweise zur Bildverarbeitung ausgebildet. Dies kann die Bereitstellung von Ansichten mit unterschiedlichen Vergrößerungen sowie ein digitales "Verschieben" eines Bildausschnitts betreffen. Diese Maßnahme hat den Vorteil, dass die Datenmenge, die zwischen der Bildaufnahmeeinheit und einer externen Bildverarbeitungseinheit bzw. Wiedergabeeinheit ausgetauscht wird, reduziert ist. Dies kann Bildverarbeitungsprozesse und die Bereitstellung bzw. Darstellung der gewünschten Bilddaten beschleunigen.

Somit kann eine (Teil-) Bildverarbeitungseinheit der Bildaufnahmeeinheit bzw. dem optischen Instrument zugeordnet sein. Gemäß dieser Ausgestaltung kann die Bildverarbeitung als verteilte Aufgabe behandelt werden, nämlich teilweise in einer internen (Teil-) Bildverarbeitungseinheit sowie einer externen (Teil-) Bildverarbeitungseinheit -jeweils aus Sicht des Instruments. Die interne (Teil-) Bildverarbeitungseinheit kann ein Teilmodul der Bildaufnahmeeinheit sein. Es ist auch vorstellbar, neben der Bildaufnahmeeinheit eine separate interne (Teil-) Bildverarbeitungseinheit beim Instrument vorzusehen.

Die Bildaufnahmeeinheit der Beobachtungsvorrichtung ist vorzugsweise als okularlose Bildaufnahmeeinheit ausgestaltet. Mit anderen Worten steht einem Bediener kein rein optischer Beobachtungsstrahlengang zur Verfügung. Stattdessen ist beispielhaft eine Aufnahmeoptik vorgesehen, an deren "Okular" der zumindest eine Bildaufnehmer angekoppelt ist. Vorzugsweise weist die Bildaufnahmeeinheit keinen optischen Zoom auf. Auf diese Weise kann die Bildaufnahmeeinheit sehr kompakt gestaltet sein. Insbesondere kann die Bildaufnahmeeinheit als Teil eines optischen Instruments, etwa eines Exoskops oder Endoskops, gestaltet sein. Vorzugsweise ist die Bildaufnahmeeinheit am distalen Ende des optischen Instruments angeordnet. Ferner ist die Bildaufnahmeeinheit vorzugsweise mit einem Beleuchtungsmodul versehen oder mit diesem gekoppelt.

Vorzugsweise ist die Wiedergabeeinheit zur unmittelbaren Wiedergabe (augenblickliche oder quasi-augenblickliche Wiedergabe) aufgenommener Bilder ausgebildet. In einer bevorzugten Ausgestaltung ist die Bildaufnahmeeinheit als Stereo-Bildaufnahmeeinheit gestaltet. Demgemäß ist dann vorzugsweise auch die Wiedergabeeinheit zur Darstellung stereoskopischer Bilder ausgerüstet. Zu diesem Zweck kann die Wiedergabeeinheit etwa zur Darstellung zweier (Stereo-)Kanäle ausgebildet sein. Ein Beobachter oder Bediener kann beispielsweise durch Nutzung geeigneter Hilfsmittel (3D-Brillen oder dgl.) das durch die Wiedergabeeinheit bereitgestellte Bild räumlich wahrnehmen. Andere Varianten sind denkbar, etwa die Bereitstellung autostereoskopischer Bildschirme. Es versteht sich, dass neben der stereoskopischen Darstellung auch grundsätzlich eine (volumetrische) 3D-Darstellung vorstellbar ist.

Im Rahmen dieser Offenbarung geht es jedoch nicht um die Darstellung von Modellen, sondern um eine unmittelbare Wiedergabe (Live-Wiedergabe) eines beobachteten Objektfeldes. Unter einer unmittelbaren Wiedergabe ist zu verstehen, dass für den Nutzer (zum Beispiel den Chirurgen) bei der Wiedergabe eines beobachteten Objekts kein zeitlicher Versatz spürbar ist.

Die Bildverarbeitungseinheit kann grundsätzlich als zentrale Einheit gestaltet sein. Es ist jedoch gemäß zumindest einigen beispielhaften Ausgestaltungen vorgesehen, dass die Bildverarbeitungseinheit als verteilte Bildverarbeitungseinheit ausgestaltet ist. Mit anderen Worten kann die Bildverarbeitungseinheit Module umfassen, von denen zumindest eines mit der Bildaufnahmeeinheit gekoppelt bzw. in dieser enthalten ist. Mit anderen Worten kann auch das optische Instrument, das mit der Bildaufnahmeeinheit versehen ist, zur Bearbeitung oder Verarbeitung von erfassten (Roh-)Bilddaten ausgestaltet sein. So ist es vorstellbar, dass der zumindest eine Bildaufnehmer einen kontinuierlichen oder quasikontinuierlichen Aufnahmebilddatenstrom bereitstellt, wobei bereits im optischen Instrument eine Wahl bzw. Ableitung eines Wiedergabebilddatenstroms erfolgt, oder zumindest eines Stroms vorbearbeiteter Bilddaten. Dies hat den Vorteil, dass keine aufwendige (separate) Rechentechnik zwischen der Bildaufnahmeeinheit und der Wiedergabeeinheit vorgesehen sein muss. Gleichwohl ist es selbstverständlich vorstellbar, dass entsprechende Bildverarbeitungsmodule vorgesehen sind, die nicht direkt der Bildaufnahmeeinheit zugeordnet sind. Derartige Bildverarbeitungsmodule können der Wiedergabeeinheit zugeordnet sein. Es ist jedoch auch vorstellbar, separate Bildverarbeitungsmodule vorzusehen, die einem Computer zugeordnet sind.

Vorzugsweise ist das Mehrachs-Eingabemodul mittelbar oder unmittelbar sowohl mit der Bildaufnahmeeinheit als auch mit der Wiedergabeeinheit und/oder der Bildverarbeitungseinheit koppelbar, um auf die verschiedenen Komponenten der Beobachtungsvorrichtung einwirken zu können. Es ist also nicht erforderlich, für die Bildaufnahmeeinheit, die Wiedergabeeinheit und ggf. sogar für die Bildverarbeitungseinheit separate Bedienmodule zur Steuerung der gewünschten Funktionen bereitzustellen.

Die genannten Komponenten können über diskrete Leitungen jeweils miteinander gekoppelt sein. Es versteht sich jedoch auch, dass eine gemeinsame Busleitung nutzbar ist. Andere Arten der Kommunikation bzw. einer Netzwerktopologie zwischen den Komponenten sind denkbar. Es versteht sich, dass zumindest einige der Komponenten auch drahtlos miteinander kommunizieren können.

Vorzugsweise entspricht die Wiedergabepixelmenge einer nativen Auflösung einer Anzeige der Wiedergabeeinheit. Sofern die Bildaufnahmeeinheit mit einem Bildaufnehmer versehen ist, der eine Vielzahl von Einzelsensoren aufweist, so dass sich insgesamt eine Aufnahmepixelmenge ergibt, die größer als die Wiedergabepixelmenge ist, kann in einfacher Weise eine Vergrößerung bereitgestellt werden, wenn, im Falle einer vergrößerten Ansicht eines Teilausschnitts eines Aufnahmebildes, jedes benachbarte Pixel des Ausschnitts ausgelesen wird. Eine (verkleinerte) Übersichtsansicht kann hingegen genutzt werden, wenn nicht jedes benachbarte Pixel, sondern etwa jedes zweite oder jedes vierte benachbarte Pixel benutzt wird, um das Wiedergabebild vom Aufnahmebild abzuleiten. Es versteht sich, dass Zwischenstufen denkbar sind. Beispielhaft kann ein Bildsensor genutzt werden, der eine 4K Auflösung (4096 × 2160 Pixel) oder eine vergleichbare Auflösung bereitstellt, wobei die Wiedergabe mit einer HD Auflösung erfolgt (z.B. Full HD 1920 × 1080 Pixel oder HD ready 1280 × 720 Pixel).

Gemäß einer beispielhaften Ausgestaltung der Beobachtungsvorrichtung ist das Eingabemodul mit der Bildaufnahmeeinheit und der Bildverarbeitungseinheit gekoppelt, um zur Variation von Bildaufnahmeparametern auf die Bildaufnahmeeinheit sowie zur Variation von Wiedergabeparametern auf die Bildverarbeitungseinheit einzuwirken, wobei das Eingabemodul vorzugsweise zumindest in einem ersten Betriebsmodus und einem zweiten Betriebsmodus betreibbar ist, wobei im ersten Betriebsmodus eine direkte Steuerung von Bildaufnahmeparametern und Wiedergabeparametern möglich ist, und wobei im zweiten Betriebsmodus eine Steuerung peripherer Funktionen ermöglicht ist. Die Kopplung kann mittelbar oder unmittelbar erfolgen. Zwischen der Bildaufnahmeeinheit und der Bildverarbeitungseinheit kann eine weitere Einheit angeordnet sein.

Gemäß der Erfindung ist ferner ein Positionierantrieb für die Bildaufnahmeeinheit vorgesehen, wobei das Eingabemodul, im zweiten Betriebsmodus, steuerbar ist, um den Positionierantrieb zur Positionierung der Bildaufnahmeeinheit zu steuern. Zu diesem Zweck kann die Bildaufnahmeeinheit an einem Stativ, einer Säule, oder einer gestellartigen Struktur aufgenommen werden, das/die zumindest zwei angetriebene Positionsachsen aufweist. Da das Eingabemodul beispielhaft als Mehrachs-Eingabemodul gestaltet ist, kann ein und dasselbe Element betätigt werden, um zwei, drei oder sogar mehr angetrieben Achsen des Positionierantriebs zu steuern.

Auf diese Weise können weitere periphere Funktionen durch das Eingabemodul gesteuert werden. Hierbei kann es sich etwa um eine Menüsteuerung oder dgl. handeln. Ferner kann das Eingabemodul dazu genutzt werden, eine Drehorientierung (Bildausrichtung) zu beeinflussen. Demgemäß kann das Eingabemodul auf einen Aktuator einwirken, der der Bildaufnahmeeinheit zugeordnet ist, und dazu ausgestaltet ist, den zumindest einen Bildaufnehmer um eine Achse senkrecht zur Bildaufnehmerfläche zu drehen.

Gemäß einer weiteren Ausgestaltung der Beobachtungsvorrichtung ist das Eingabemodul als Einhand-Eingabemodul ausgestaltet, wobei das Eingabemodul ein durch einen Bediener manipulierbares Betätigungselement aufweist, das mehrere Bewegungsfreiheitsgrade für Eingaben bereitstellt, insbesondere zumindest eine Translationsrichtung, zumindest eine Rotationsrichtung und zumindest zwei weitere Freiheitsgrade bzw. Bewegungsrichtungen, die als Schubrichtungen oder Schwenkrichtungen ausgestaltet sind. Das Einhand-Eingabemodul kann mit lediglich einer Hand bedient werden. Somit wird sichergestellt, dass zumindest eine zweite Hand des Bedieners frei bleibt.

Das Eingabemodul der Erfindung weist also nicht nur zwei Freiheitsgrade in Form einer Rotationsrichtung für eine Drehbewegung sowie einer Translationsrichtung für eine Drückbewegung auf. Zumindest zwei weitere Freiheitsgrade stehen zur Verfügung, die vorzugsweise senkrecht zueinander orientiert und senkrecht zur Translationsrichtung orientiert sind.

Gemäß einer weiteren Ausgestaltung der Beobachtungsvorrichtung ist das Betätigungselement puckartig oder knaufartig gestaltet, wobei das Betätigungselement mit Sensoren gekoppelt ist, um eine Zug-/Druckbewegung entlang einer Längsachse des Betätigungselements, eine Drehbewegung um die Längsachse, und Schubbewegungen in einer Ebene, die senkrecht zur Längsachse orientiert ist oder Schwenkbewegungen um Schwenkachsen, die senkrecht zur Längsachse orientiert sind, zu erfassen. Vorzugsweise stellt das Betätigungselement eine Handauflage bzw. Handtellerauflage bereit. Somit kann der Bediener in einfacher Weise seine Hand auf dem Betätigungselement ablegen und dieses zumindest abschnittsweise mit seinen Fingern umgreifen. Es versteht sich, dass Ausgestaltungen des Betätigungselements denkbar sind, bei denen der Bediener lediglich mit seinen Fingern an dieses angreift, ohne den Handteller darauf abzulegen.

Gemäß einer weiteren Ausgestaltung der Beobachtungsvorrichtung ist das Betätigungselement mit zumindest einem als Wegaufnehmer oder Kraftaufnehmer ausgebildeten Sensor gekoppelt. Vorzugsweise ist das Betätigungselement mit einer Mehrzahl von Sensoren zur multiaxialen Erfassung von Verformungen oder Bewegungen gekoppelt.

Es sind verschiedene Arten von Sensoren denkbar. Beispielhaft kann es sich bei den Sensoren um optische Sensoren handeln, die Bewegungen des Betätigungselements erfassen können. In diesem Zusammenhang wird beispielhaft auf optische Sensoren von Computer-Mäusen verwiesen.

Es ist jedoch auch vorstellbar, das Betätigungselement als solches nicht bewegbar zu gestalten. Stattdessen können Sensoren vorgesehen sein, die (minimale) Verformungen des Betätigungselements erfassen können. Mit anderen Worten kann ein Bediener das Betätigungselement manipulieren, etwa stauchen, ziehen, tordieren und/oder biegen, wobei die dabei entstehenden Deformationen durch geeignete Sensoren erfasst werden. Beispielhaft können zu diesem Zweck Dehnmessstreifen oder ähnliche Sensoren vorgesehen sein.

Vorzugsweise werden zwei, drei oder mehr Sensoren verwendet, um eine entsprechende Anzahl an Bewegungsfreiheitsgraden bereitzustellen bzw. eine entsprechende Anzahl definierter Betätigungen erfassen und voneinander unterscheiden zu können. Es versteht sich, dass die Sensoren dazu ausgestaltet sein können, gleichzeitig mehrere Bewegungen (kombinierte Betätigungsbewegungen) zu erfassen. Dies kann beispielsweise ein gleichzeitiges Ziehen oder Drücken verbunden mit einem Drehen des Betätigungselements umfassen. Auf diese Weise können weitere Funktionen mit lediglich einem Betätigungselement gesteuert werden.

Das Eingabemodul kann neben dem Betätigungselement weitere Eingabeelemente aufweisen, etwa Knöpfe, Taster, Scroll-Räder oder dgl. Jedoch ist es bevorzugt, wenn das Betätigungselement selbst nicht mit weiteren Eingabeelementen versehen ist. Diese können vielmehr im Umfeld des Betätigungselements angeordnet sein. Somit kann das Betätigungselement "blind" bedient werden, ohne dass ein Sichtkontakt erforderlich ist. Gleichwohl sind in einigen beispielhaften Ausgestaltungen Bestätigungstaster oder Bestätigungsschalter direkt am Betätigungselement zur Bestätigung von Bedienereingaben denkbar.

Gemäß einer weiteren Ausgestaltung der Beobachtungsvorrichtung ist das Betätigungselement als Vier-Achs-Betätigungselement gestaltet, wobei eine Betätigung der ersten Betätigungsachse eine Vergrößerung und eine der Vergrößerung zugeordnete Größe eines Bereichs der Wiedergabebilder in den Aufnahmebildern definiert, wobei eine Betätigung der zweiten Betätigungsachse eine Fokuseinstellung definiert, wobei eine Betätigung der dritten Betätigungsachse eine Bewegung des von den Wiedergabebildern abgedeckten Bereichs in einem durch die Aufnahmebilder abgedeckten Bereich in einer ersten Bewegungsrichtung bewirkt, und wobei eine Betätigung der vierten Betätigungsachse eine Bewegung des von den Wiedergabebildern abgedeckten Bereichs in dem durch die Aufnahmebilder abgedeckten Bereich in einer zweiten Bewegungsrichtung bewirkt, die zur ersten Bewegungsrichtung geneigt ist. Demgemäß weist das Betätigungselement vier (Bewegungs-)Freiheitsgrade auf, denen die Betätigungsachsen zugeordnet sind.

Vorzugsweise sind die erste Bewegungsrichtung und die zweite Bewegungsrichtung senkrecht zueinander orientiert.

Die Fokuseinstellung kann insbesondere die Variation oder Verstellung einer Fokusebene bewirken. Zu diesem Zweck werden üblicherweise optische Bauteile der Bildaufnahmeeinheit verfahren. Die Bildaufnahmeeinheit weist dann einen Fokusantrieb auf. Ferner kann die Fokuseinstellung eine Variation der Tiefenschärfe betreffen. Die Betätigung der ersten Betätigungsachse, etwa durch Drücken oder Ziehen, bewirkt eine (digitale) Vergrößerung bzw. Verkleinerung des Bildausschnitts, der durch die Wiedergabeeinheit angezeigt wird. Eine Betätigung der dritten bzw. der vierten Betätigungsachse bewirkt eine Bewegung des Bildausschnitts, der gerade angezeigt wird, in einer bereitgestellten Bildebene. Es versteht sich, dass eine derartige Bewegung nicht möglich ist, wenn der Bereich, der durch die Wiedergabeeinheit gerade angezeigt wird, dem Bereich entspricht, der durch die Bildaufnahmeeinheit bereitgestellt wird.

Der von den Wiedergabebildern abgedeckte Bereich kann auch als Wiedergabebereich bezeichnet werden. Der durch die Aufnahmebilder bereitgestellte Bereich kann auch als Aufnahmebereich bezeichnet werden.

Gemäß einer beispielhaften Weiterbildung stellt die erste Betätigungsachse eine Translationsrichtung bereit, wobei die zweite Betätigungsachse eine Rotationsrichtung bereitstellt, deren Achse parallel zur Translationsrichtung ist, und wobei die dritte Betätigungsachse und die vierte Betätigungsachse jeweils eine Schubrichtung, zur Erfassung einer seitlichen Auslenkung senkrecht zur Translationsrichtung, oder eine Schwenkrichtung, zur Erfassung einer seitlichen Neigung um zur Translationsachse senkrecht orientierte Achsen, bereitstellen. Es versteht sich, dass auch eine Kombination aus einer Schwenkrichtung und einer Schubrichtung vorstellbar ist.

Auf diese Weise kann durch lediglich ein Betätigungselement, das mit einer Hand bedienbar ist, eine Vielzahl von Funktionen gesteuert werden.

Gemäß einer weiteren Ausgestaltung der Beobachtungsvorrichtung beträgt die Aufnahmepixelmenge ein ganzzahliges Vielfaches der Wiedergabepixelmenge, wobei die Aufnahmepixelmenge vorzugsweise ein Vierfaches, weiter bevorzugt ein Achtfaches der Wiedergabepixelmenge beträgt. Beispielhaft beträgt die Wiedergabepixelmenge 1280 × 720 (HD), vorzugsweise 1920 × 1080 (Full HD). Demgemäß kann die Aufnahmepixelmenge etwa ein Zweifaches, Vierfaches oder gar Achtfaches dieser Werte betragen, wobei vorzugsweise das Seitenverhältnis (etwa 16:9 oder 4:3) gewahrt bleibt.

Gemäß einer weiteren Ausgestaltung ist die Bildverarbeitungseinheit dazu ausgebildet, der Wiedergabeeinheit eine interpolationsfrei darstellbare Wiedergabepixelmenge bereitzustellen. Vorzugsweise umfasst dies eine Definition der Wiedergabepixelmenge, bei der jedes Wiedergabepixel einem Pixel der Aufnahmepixelmenge oder einem definierten Mittelwert eines Satzes von Pixeln (etwa 2 × 2 oder 4 × 4) der Aufnahmepixelmenge entspricht. Mit anderen Worten erlaubt die Beobachtungsvorrichtung zumindest in einigen Vergrößerungsstufen einen verlustfreien oder quasi-verlustfreien Digital-Zoom. Es versteht sich, dass auch Zwischenstufen denkbar sind, bei denen eine Interpolation erforderlich ist. Es versteht sich auch, dass, im Falle einer starken Vergrößerung, der zu vergrößernde Ausschnitt des Aufnahmebereichs weniger Pixel aufweisen kann als der Wiedergabebereich, so dass dann ein "Hochskalieren" auf das Format des Wiedergabebereichs erfolgt. Somit können zumindest einige Vergrößerungsstufen einen verlustbehafteten Digital-Zoom umfassen.

Gleichwohl ermöglicht diese Gestaltung einen Verzicht auf einen aufwendigen optischen Zoom bei der Bildaufnahmeeinheit. Der Digital-Zoom hat den weiteren Vorteil, dass auch bei der Variation der Vergrößerung bzw. des aktuell durch die Wiedergabeeinheit angezeigten Bildausschnitts die Beleuchtungsverhältnisse und auch die Kontrasteigenschaften des Gesamtbildes konstant bleiben. Dies führt wiederum zu Vereinfachungen auf Seiten der Bildaufnahmeeinheit.

Gemäß einer weiteren Ausgestaltung der Beobachtungsvorrichtung ist die Bildaufnahmeeinheit dazu ausgebildet, Bilddaten einer Rohdatenpixelmenge zu erfassen, die größer als die Aufnahmepixelmenge ist, wobei die Rohdatenpixelmenge einem Gesamterfassungsbereich des Bildaufnehmers entspricht, und wobei die Aufnahmepixelmenge als Ausschnitt der Rohdatenpixelmenge gewählt ist. Diese Ausgestaltung hat den Vorteil, dass ein Randbereich des Gesamterfassungsbereichs nicht für die Bereitstellung des Aufnahmebereichs gewählt werden muss. Im Randbereich von Bildaufnehmern treten häufig nachteilige optische Effekte auf, etwa Verzerrungen, Kontrastabweichungen, Unschärfen oder dgl. Insofern ist es von Vorteil, wenn auf die Weiterverarbeitung dieses Bereichs verzichtet wird.

Ein weiterer Vorteil der oben genannten Ausgestaltung kann sich bei Nutzung einer zur Stereoerfassung ausgebildeten Bildaufnahmeeinheit ergeben, die zwei entsprechende Bildaufnehmer bereitstellt. Wenn der Gesamterfassungsbereich zumindest eines der beiden Bildaufnehmer größer als der jeweils als Aufnahmebereich gewählte Bereich ist, kann der seitliche Abstand zwischen den beiden Halbbildern, die schlussendlich das Stereobild bilden, variiert werden. Dies kann von Vorteil für die stereoskopische Darstellung sein. Zur Variation des Versatzes zwischen den Halbbildern ist keine mechanische oder optische Manipulation erforderlich. Vielmehr kann der jeweilige Aufnahmebereich im Gesamterfassungsbereich entsprechend verschoben werden.

Gemäß einer weiteren Ausgestaltung der Beobachtungsvorrichtung ist die Bedieneinheit dazu ausgebildet, am Eingabemodul eine haptische Rückmeldung bereitzustellen, insbesondere beim Erreichen von Grenzwerten oder Extremwerten von Parameterbereichen, die über das Eingabemodul steuerbar sind.

Auf diese Weise kann das Eingabemodul beispielhaft eine sog. Force Feedback-Funktion aufweisen. So ist es vorstellbar, Vibrationsgeber oder ähnliche Aktuatoren am Eingabemodul vorzusehen, und insbesondere mit dem Betätigungselement zu koppeln. Die haptische Rückmeldung hat den Vorteil, dass die Rückmeldung über den Tastsinn des Bedieners erfolgen kann, nämlich direkt an die Hand, mit der der Bediener das Eingabemodul betätigt. Auf diese Weise erfolgt keine wesentliche optische und/oder akustische Ablenkung.

Beispielhaft kann die haptische Rückmeldung genutzt werden, um dem Bediener zu signalisieren, dass er eine maximale Zoom-Stufe erreicht hat. Ein weiteres Beispiel für eine haptische Rückmeldung ist das Erreichen eines Randbereichs des Aufnahmebereichs beim "Verschieben" des Wiedergabebereichs im Aufnahmebereich.

Gemäß der Erfindung ist die Bildverarbeitungseinheit dazu ausgebildet, Übersichtsbilder zu erzeugen, die einen Bereich repräsentieren, der im Wesentlichen einem durch die Aufnahmepixelmenge umfassten Bereich entspricht, wobei die Übersichtsbilder eine Übersichtspixelmenge aufweisen, die kleiner als die Wiedergabepixelmenge gewählt ist, wobei die Übersichtsbilder durch die Wiedergabeeinheit zumindest zeitweise parallel zu den Wiedergabebildern darstellbar sind, und wobei die Übersichtsbilder die Wiedergabebilder abschnittsweise überdecken. Auf diese Weise kann etwa eine Bild-in-Bild Funktion bewirkt werden. Somit kann die visuelle Navigation vereinfacht werden. Die Übersichtsbilder können teiltransparent gestaltet sein. Es versteht sich, dass die Übersichtsbilder nicht ständig eingeblendet werden müssen. So ist es vorstellbar, die Übersichtsbilder dann einzublenden, wenn der Bediener aktuell am Bedienmodul eine Eingabe vornimmt. Dies kann etwa ein Verschieben des Wiedergabebereichs oder eine Änderung der Vergrößerung betreffen. In diesen Situationen ist es von Vorteil, das Übersichtsbild einzublenden.

Es versteht sich, dass am Eingabemodul separate Eingabeelemente ausgebildet sein können, etwa Knöpfe, Schalter oder dgl., um das Übersichtsbild bei Bedarf einzublenden und auszublenden.

Gemäß der Erfindung ist die Wiedergabeeinheit dazu ausgebildet, in den dargestellten Übersichtsbildern einen Ausschnittsbereich hervorzuheben, der einem durch die Wiedergabepixelmenge umfassten Bereich innerhalb der Aufnahmepixelmenge entspricht, wobei sich der Ausschnittsbereich in den Übersichtsbildern bewegt, wenn durch Betätigung des Bedienelements der durch die Wiedergabepixelmenge abgedeckte Bereich im durch die Aufnahmepixelmenge abgedeckten Bereich bewegt wird. Auf diese Weise kann die Positionierung noch weiter vereinfacht werden.

Gemäß einer weiteren Ausgestaltung der Beobachtungsvorrichtung sind zumindest die Bildaufnahmeeinheit und das Eingabemodul der Bedieneinheit autoklavierbar. Auf diese Weise kann in einfacher Weise eine Sterilisation dieser Komponenten durchgeführt werden. Dies ist insbesondere im Umfeld von medizinischen Eingriffen von wesentlichem Vorteil.

Das Eingabemodul betreffend ist es bei zumindest einigen beispielhaften Ausgestaltungen vorgesehen, die Erfassung der Betätigungsbewegungen durch Erfassung von Verformungen oder Deformationen des Betätigungselements zu bewerkstelligen. Demgemäß kann das mit dem Betätigungselement versehene Eingabemodul integral gestaltet sein und vorzugsweise nur wenige separat zueinander bewegbare Bauteile aufweisen. Auf diese Weise ist das Eingabemodul besonders unempfindlich gestaltet und geeignet für diverse Sterilisationsverfahren.

Eine weitere Ausgestaltung bzw. Betriebsart der Beobachtungsvorrichtung umfasst die Zuordnung einer Aktion, insbesondere einer zu steuernden Funktion, zu mehreren Freiheitsgraden bzw. Betätigungsachsen des Betätigungselements. Zum Beispiel kann eine Zoom-Funktion sowohl durch ein Verdrehen (Nutzung der Rotationsrichtung) als auch durch eine Zug-/Druckbewegung (Nutzung der Translationsrichtung) gesteuert werden. Auf diese Weise wird eine redundante Bedienung ermöglicht. So ist es vorstellbar, die zugeordneten Freiheitsgrade mit definierter Hierarchie oder Priorisierung der Funktion zuzuordnen, so dass die Bedienung weiterhin eindeutig und intuitiv erfolgen kann. Dies kann etwa eine zeitliche Änderung der Zuordnung betreffen. Ferner ist es vorstellbar, auf diese Weise eine Grobverstellung und Feinverstellung zu verwirklichen, wobei ein Freiheitsgrad der Grobverstellung und ein anderer Freiheitsgrad der Feinverstellung zugeordnet ist. Dies kann etwa die Zoom-Funktion oder den Fokusantrieb betreffen. Generell können sich die verschiedenen Freiheitsgrade im Hinblick auf die Feinfühligkeit der Verstellung/Betätigung unterscheiden.

Eine weitere Ausgestaltung bzw. Betriebsart der Beobachtungsvorrichtung umfasst die Definition von Vorzugsfunktionen, wobei zugeordnete Betätigungen entsprechend hoch priorisiert erfasst und ausgewertet werden. Niedrig priorisierte (nachrangige) Betätigungen, die aktuell nicht bevorzugten Funktionen zugeordnet sind, können hierbei ignoriert werden. Mit anderen Worten können bestimmte Funktionen gesperrt sein, so dass über die entsprechenden Freiheitsgrade eingeleitete Betätigungen nicht zu entsprechenden Aktionen führen. Dies kann bestimmte Aktionen vereinfachen, da unbeabsichtigte Betätigungen anderer Funktionen vermieden werden können. So kann zum Beispiel in bestimmten Betriebsarten eine sichere Entkopplung der Zoom-Funktion vom Fokusantrieb bzw. von einer Verschiebung des Bildausschnitts erfolgen.

Gemäß einer weiteren beispielhaften Ausgestaltung der Beobachtungsvorrichtung weist die Bedieneinheit eine Montageschnittstelle auf, die insbesondere als Teil einer Schelllösekupplung (Quick-Release Kupplung) gestaltet ist, um die Bedieneinheit lösbar mit einer Halterung oder einem Stativ zu verbinden. Ferner kann zwischen der Bildaufnahmeeinheit und dem Stativ oder der Halterung eine ähnliche Montageschnittstelle ausgebildet sein. Somit können der Bildaufnahmeeinheit und die Bedieneinheit einfach, sicher und lösbar an ein und demselben Stativ oder ein und derselben Halterung, oder an verschiedenen Stativen oder Halterungen aufgenommen sein.

Gemäß einer weiteren beispielhaften Ausgestaltung der Beobachtungsvorrichtung ist die Bedieneinheit dazu ausgestaltet, mit einer sterilen Hülle überzogen zu werden, wobei die Bedieneinheit ferner dazu ausgestaltet ist, durch die sterile Hülle hindurch bedient zu werden.

Ferner bezieht sich die vorliegende Offenbarung allgemein auf eine Verwendung eines als Einhand-Eingabemodul ausgestalten Mehrachs-Eingabemoduls bei einer Bedieneinheit einer medizinischen Beobachtungsvorrichtung zur Steuerung von Bildaufnahmeparametern und Wiedergabeparametern und zur Steuerung eines Positionierantriebs zur Positionierung der Bildaufnahmeeinheit, wobei das Eingabemodul zumindest in einem ersten Betriebsmodus und einem zweiten Betriebsmodus betreibbar ist, wobei im ersten Betriebsmodus eine direkte Steuerung von Bildaufnahmeparametern und Wiedergabeparametern ermöglicht ist, wobei das Eingabemodul im zweiten Betriebsmodus steuerbar ist, um den Positionierantrieb zu steuern, wobei das Eingabemodul ein durch einen Bediener manipulierbares Betätigungselement aufweist, das mehrere Bewegungsfreiheitsgrade für Eingaben bereitstellt, und wobei verschiedene Achsen des Positionierantriebs gleichzeitig durch das Eingabemodul steuerbar sind.

Die Verwendung betreffend bezieht sich die Erfindung ferner auf die Verwendung eines Mehrachs-Eingabemoduls. insbesondere eines Einhand-Eingabemoduls, bei einer Bedieneinheit einer außerhalb des Patientenkörpers befindlichen medizinischen Beobachtungsvorrichtung zur Steuerung von Bildaufnahmeparametern und Wiedergabeparametern, wobei die Beobachtungsvorrichtung eine Bildaufnahmeeinheit zur Bereitstellung von Aufnahmebildern einer vordefinierten Aufnahmepixelmenge und eine Wiedergabeeinheit zur Darstellung von Wiedergabebildern einer vordefinierten Wiedergabepixelmenge aufweist, wobei das Eingabemodul eine Mehrzahl von Betätigungsachsen aufweist, von denen eine Betätigungsachse zur Wahl eines Vergrößerungsmodus und zumindest zwei Betätigungsachsen zur Bewegung eines der Wiedergabepixelmenge, bei Beachtung eines aktuellen Vergrößerungsmodus, entsprechenden Bereichs in einem der Aufnahmepixelmenge entsprechenden Bereich nutzbar sind.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1: eine schematische Seitenansicht einer Ausführungsform einer Beobachtungsvorrichtung, welche nicht Teil der Erfindung ist;
- Fig. 2: eine perspektivische Draufsicht einer Bildaufnahmeeinheit, die in Form eines Exoskops gestaltet ist;
- Fig. 3: eine weitere perspektivische Ansicht der Anordnung gemäß Fig. 2 in abgewandelter Orientierung;
- Fig. 4: eine perspektivische Draufsicht einer Ausgestaltung eines Einhand-Eingabemoduls;
- Fig. 5: eine perspektivische Draufsicht einer weiteren Ausgestaltung eines EinhandEingabemoduls;
- Fig. 6: eine perspektivische Ansicht noch einer weiteren Ausgestaltung eines EinhandEingabemoduls;
- Fig. 7: eine schematische Darstellung eines Aufnahmebildes;
- Fig. 8: eine schematische Darstellung eines Wiedergabebildes;
- Fig. 9: eine schematische Darstellung eines Wiedergabebildes, das einen Bereich abdeckt, der einem Aufnahmebildbereich entspricht;
- Fig. 10: eine schematische Darstellung eines Wiedergabebildes, das zu Veranschaulichungszwecken einem Aufnahmebild überlagert ist, wobei ein Wiedergabebildbereich einen Teil des Aufnahmebildbereichs abdeckt;
- Fig. 11: eine weitere Darstellung gemäß Fig. 10, wobei das Wiedergabebild einen Wiedergabebereich im Aufnahmebereich des Aufnahmebilds abdeckt, dessen Pixelmenge kleiner als die Pixelmenge des Wiedergabebereichs ist;
- Fig. 12: eine weitere Darstellung eines Aufnahmebildes, wobei ein Gesamterfassungsbereich angedeutet ist, der größer als ein Aufnahmebereich des Aufnahmebildes ist;
- Fig. 13: eine schematische Darstellung eines Eingabemoduls und eines Wiedergabebildes, das einem Aufnahmebild überlagert ist, zur Veranschaulichung einer Positionssteuerung;
- Fig. 14: eine weitere, an Fig. 13 angelehnte Darstellung zur Veranschaulichung einer Zoom-Funktion;
- Fig. 15: eine schematische vereinfachte Darstellung eines Eingabemoduls und einer Bildaufnahmeeinheit zur Veranschaulichung einer Fokusverstellung;
- Fig. 16: eine weitere schematische Gegenüberstellung eines Aufnahmebildes und eines Wiedergabebildes zur Veranschaulichung einer Bild-in-Bild Funktion;
- Fig. 17: eine schematische Seitenansicht einer weiteren Ausführungsform einer Beobachtungsvorrichtung, welche Teil der Erfindung ist; und
- Fig. 18: eine schematische Seitenansicht einer weiteren Ausführungsform eines EinhandEingabemoduls, das mit einer sterilen Hülle versehen ist.

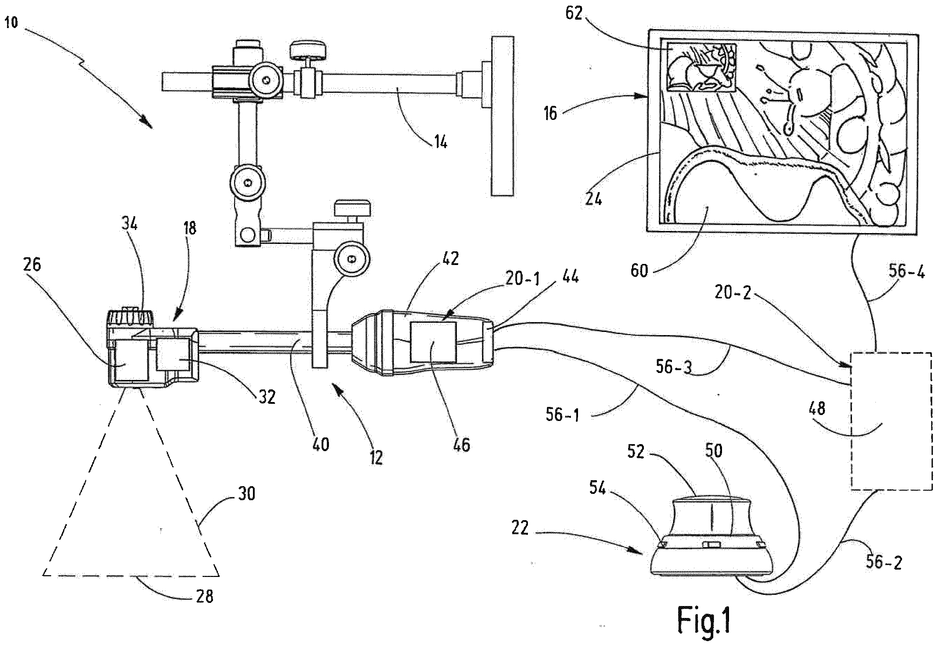

Fig. 1 zeigt in schematischer Darstellung eine beispielhafte Konfiguration einer Beobachtungsvorrichtung 10, die nicht Teil der Erfindung ist. Die Beobachtungsvorrichtung 10 umfasst ein optisches Instrument 12. Das optische Instrument ist beispielhaft als Exoskop ausgestaltet. Es sind alternative Ausführungsformen vorstellbar, bei denen das optische Instrument als Endoskop gestaltet ist. Das optische Instrument 12 ist beispielhaft an einem Stativ oder Ständer 14 aufgenommen, der auch als Haltearm bezeichnet werden kann. Demgemäß ergibt sich eine fixe Orientierung des optischen Instruments 12 in Bezug auf ein zu beobachtendes Objekt, etwa ein Körperteil oder ein Organ eines Patienten.

Die Beobachtungsvorrichtung 10 weist ferner eine Wiedergabeeinheit 16 auf, die zumindest eine Anzeige bzw. einen Bildschirm 24 umfasst. Sowohl das optische Instrument 12 als auch die Wiedergabeeinheit 16 können als stereoskopische Geräte gestaltet sein. Demgemäß kann das optische Instrument 12 dazu ausgestaltet sein, rechte und linke Halbbilder aufzunehmen, wobei die Wiedergabeeinheit 16 dazu ausgebildet ist, die rechten und linken Halbbilder in gewünschter Weise darzustellen, um einen stereoskopischen (räumlichen) Eindruck beim Sehen hervorzurufen. Im Regelfall umfasst jedes Halbbild eine vollständige Darstellung des beobachteten Objektes, wobei zwischen den Darstellungen der rechten und linken Halbbilder ein Versatz oder Versatzwinkel vorliegen kann. Auf diese Weise kann sich der räumliche Eindruck ergeben. Die Wiedergabeeinheit 16 kann ferner sog. 3D-Brillen, HMD-Brillen (head-mounted display) und ähnliche Einrichtungen aufweisen.

Das optische Instrument 12 weist eine Bildaufnahmeeinheit 18 auf. Beispielhaft ist die Bildaufnahmeeinheit 18 in das optische Instrument 12 integriert und vorzugsweise an dessen distalem Ende angeordnet. Ferner umfasst die Beobachtungsvorrichtung 10 eine Bildverarbeitungseinheit 20. Die Bildverarbeitungseinheit 20 kann grundsätzlich als zentrale Bildverarbeitungseinheit oder als dezentrale (verteilte) Bildverarbeitungseinheit gestaltet sein. Beispielhaft veranschaulicht Fig. 1 eine Ausgestaltung, bei der die Bildverarbeitungseinheit 20 Abschnitte 20-1 und 20-2 aufweist. Die (Teil-) Bildverarbeitungseinheit 20-1 ist dem optischen Instrument 12 zugeordnet. Die (Teil-) Bildverarbeitungseinheit 20-2 ist beispielhaft separat dargestellt und ist hier als separate Komponente ausgeführt sein. Es versteht sich, dass die (Teil-) Bildverarbeitungseinheit 20-2 auch zumindest teilweise in die Wiedergabeeinheit 16 (zumindest baulich) integriert sein kann.

Allgemein ist die Bildverarbeitungseinheit 20 der Bildaufnahmeeinheit 18 und der Wiedergabeeinheit 16 zwischengeordnet, um Bilddaten, die durch die Bildaufnahmeeinheit 18 bereitgestellt werden, zu verarbeiten und aufzubereiten, um diese der Wiedergabeeinheit 16 zur Wiedergabe zuzuführen.

Die Bildaufnahmeeinheit 18 umfasst zumindest einen Bildaufnehmer 26, der dazu ausgestaltet ist, eine definierte Objektebene 28 zu beobachten und die erfassten optischen Signale in elektrische (Daten-)Signale zu überführen. Ein zweiter Bildaufnehmer (in Fig. 1 nicht explizit dargestellt) erlaubte eine stereoskopische Darstellung. Ein Sichtfeld des Bildaufnehmers 26 ist in Fig. 1 mit 30 angedeutet. Die Objektebene 28 ist bei dem beispielhaft als Exoskop ausgeführten optischen Instrument 12 gemäß Fig. 1 vom Bildaufnehmer 26, insbesondere von einem Objektiv des Bildaufnehmers 26, beabstandet. Ein Exoskop wird üblicherweise mit einem Objektabstand (Arbeitsabstand) von ungefähr 250 mm bis ungefähr 750 mm verwendet. Der Bildaufnehmer 26 umfasst beispielsweise einen CCD Sensor.

Das in Fig. 1 dargestellte optische Instrument 12 ist nicht dafür ausgebildet, in Körperöffnungen oder sonstige engen Stellen eingeführt zu werden. Gleichwohl weist die Beobachtungsvorrichtung 10 gemäß alternativer Ausgestaltungen ein Instrument 12 auf, das als endoskopisches Instrument gestaltet ist und zur Einführung in Körperöffnungen ausgebildet ist.

Die Bildaufnahmeeinheit 18 umfasst ferner eine Beleuchtungseinheit 32. Die Beleuchtungseinheit 32 kann in das Instrument 12 integriert sein. Es ist jedoch auch vorstellbar, externe Lichtquellen an das Instrument 12 anzukoppeln, um im Bereich der Bildaufnahmeeinheit 18 eine Beleuchtung für die Objektebene 28 bereitzustellen.

Ferner ist der Bildaufnahmeeinheit 18 eine Aufrichteinheit oder Bildaufrichtung 34 zugeordnet. Bei der Bildaufrichtung 34 handelt es sich beispielhaft um eine motorisch oder manuell antreibbare Aufrichtung, die den zumindest einen Bildaufnehmer 26 um die Längsachse des Objektivs, bzw. die optische Achse, rotiert. Bei zwei Bildaufnehmern wie im vorliegenden Beispiel werden beide Bildaufnehmer 26-1 und 26-2 gemeinsam um eine Achse senkrecht zur Stereobasis rotiert, wobei die Achse mittig zwischen den Bildaufnehmern verläuft. Auf diese Weise kann die Orientierung (Drehorientierung) des aufgenommenen Bildes beeinflusst werden.

Ferner umfasst das optische Instrument 12 einen Schaft 40. Am proximalen Ende des Schaftes 40, das dem distalen Ende abgewandt ist, ist ein Griffgehäuse 42 ausgebildet. Grundsätzlich ist das optische Instrument 12 auch als handführbares Instrument ausgeführt. Demgemäß kann das Instrument 12 am Griffgehäuse 42 ergriffen und geführt werden. Gleichwohl ist die starre Aufnahme am Stativ (Haltearm) 14 für eine Vielzahl von Anwendungsfällen von Vorteil.

Ferner weist das optische Instrument 12, etwa an seinem proximalen Ende, eine Schnittstelle 44 auf, die insbesondere als Datenschnittstelle und Kommunikationsschnittstelle ausgestaltet ist. Über die Schnittstelle 44 können Bilddaten transferiert werden. Ferner kann das optische Instrument 12 über die Schnittstelle 44 mit Steuerbefehlen versorgt werden, die etwa durch die Bedieneinheit 22 erzeugt und/oder durch die externe (Teil-)Bildverarbeitungseinheit 20-2 übermittelt werden.

Die in Fig. 1 als verteilte Einheit dargestellte Bildverarbeitungseinheit 20 umfasst beispielhaft ein erstes Verarbeitungsmodul 46 und ein zweites Verarbeitungsmodul 48. Das erste Verarbeitungsmodul 46 ist dem Instrument 12 zugeordnet und mit der Bildaufnahmeeinheit 18 gekoppelt. Das zweite Verarbeitungsmodul 48 ist als externes oder separates Verarbeitungsmodul ausgeführt.

Die Beobachtungsvorrichtung 10 umfasst ferner eine Bedieneinheit 22, die nachfolgend näher beschrieben wird. Die Bedieneinheit 22 ist mittelbar oder unmittelbar mit der Bildaufnahmeeinheit 18, der Bildverarbeitungseinheit 20 und/oder der Wiedergabeeinheit 16 koppelbar.

Die Bedieneinheit 22 umfasst beispielhaft ein Eingabemodul 50, das vorzugsweise als Einhand-Eingabemodul ausgeführt ist. Das Eingabemodul 50 umfasst ein Betätigungselement 52, vorzugsweise ein Einhand-Betätigungselement. Das Betätigungselement 52 ist etwa puckartig, kugelartig oder knopfartig bzw. scheibenartig gestaltet. Vorzugsweise weist das Betätigungselement 52 eine Erstreckung auf, die es einem Bediener erlaubt, das Betätigungselement 52 mit der Hand zu umgreifen, etwa ähnlich einer Computer-Maus. Insofern weist das in Fig. 1 gezeigte Betätigungselement 52 des Eingabemoduls 50 etwa einen Durchmesser von zumindest 30 mm (Millimeter), vorzugsweise einen Durchmesser von zumindest 40 mm, weiter bevorzugt einen Durchmesser von zumindest 50 mm auf. Auf diese Weise kann das Betätigungselement 52 etwa auch bei Verwendung von Handschuhen sicher und nachvollziehbar ergriffen und betätigt werden. Beispielhaft weist das Eingabemodul 50 neben dem Betätigungselement 52 weitere Betätigungselemente 54 in Form von Tastern oder dgl. auf. Auf diese Weise können Zusatzfunktionen verwirklicht werden.

In Fig. 1 veranschaulichen ferner mit 56-1, 56-2, 56-3 und 56-4 bezeichnete Leitungen, dass die Komponenten der Beobachtungsvorrichtung 10 grundsätzlich miteinander kommunizieren können, sei es auf direktem oder auf indirektem (mittelbarem) Wege. Es versteht sich, dass zumindest einige der in Fig. 1 gezeigten Leitungen 56-1, 56-2, 56-3 und 56-4 als kabellose Verbindungen ausgeführt sein können. Darüber hinaus ist es auch vorstellbar, dass die Komponenten der Beobachtungsvorrichtung 10 an einen gemeinsamen Kommunikations-Switch ankoppeln und sich demgemäß etwa eine sternförmige Topologie ergibt. Andere Arten, etwa Bussysteme oder dgl., sind denkbar.

Beispielhaft zeigt die Anzeige 24 der Wiedergabeeinheit 16 eine Darstellung von Organen eines Patienten. Die Wiedergabeeinheit 16 ist dazu ausgestaltet, ein Wiedergabebild 60 abzubilden, das auf einem Aufnahmebild (in Fig. 1 nicht separat gezeigt) basiert, das durch die Bildaufnahmeeinheit 18 bereitgestellt wird. Dem Wiedergabebild 60 kann zumindest abschnittsweise und zumindest zeitweise ein Übersichtsbild 62 überlagert sein, etwa nach Art einer Bild-in-Bild-Darstellung. Das Übersichtsbild 62 veranschaulicht beispielhaft einen Gesamtbereich, der durch die Bildaufnahmeeinheit 18 erfassbar ist, wobei das Wiedergabebild 60, je nach gewählter Vergrößerung, den Gesamtbereich oder Teilbereiche davon zeigt. Insofern hilft das Übersichtsbild 62 bei der Orientierung bzw. Navigation.

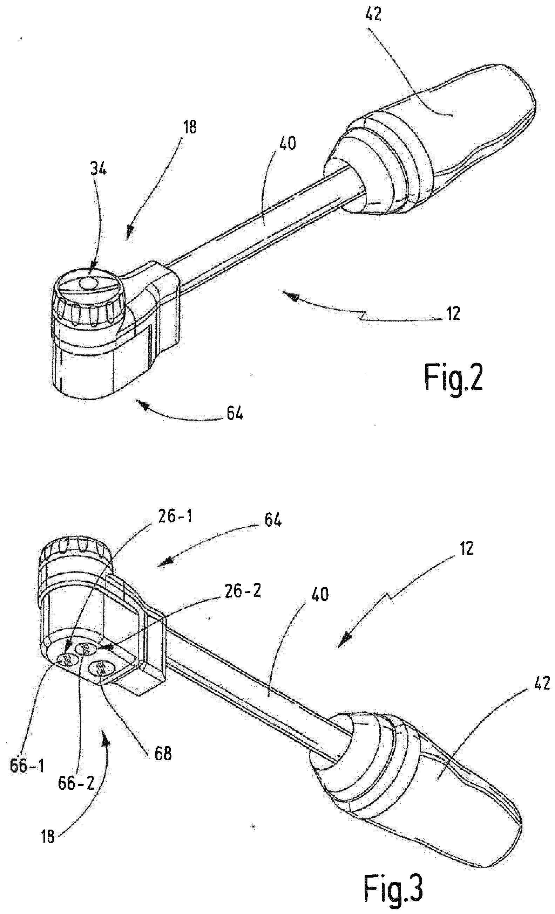

Anhand der Fig. 2 und 3 wird eine beispielhafte Ausgestaltung eines als Exoskop ausgestalteten optischen Instruments 12 näher veranschaulicht. Das in den Fig. 2 und 3 gezeigte optische Instrument entspricht in seinem Grundaufbau grundsätzlich dem optischen Instrument 12 gemäß Fig. 1.

Das optische Instrument 12 weist einen Schaft 40 auf, an dessen distalem Ende ein Aufnahmekopf 64 ausgebildet ist. Der Aufnahmekopf 64 beherbergt die Bildaufnahmeeinheit 18, zumindest Teile davon. An einem proximalen Ende des Schafts 40, das dem distalen Ende abgewandt ist, ist ein Griffgehäuse 42 ausgebildet, das weitere Komponenten des optischen Instruments 12 beherbergen kann.

Fig. 3 veranschaulicht, dass das optische Instrument 12 beispielhaft als stereoskopisches Instrument ausgestaltet sein kann. Demgemäß weist die Bildaufnahmeeinheit 18 im Bereich des Aufnahmekopfes 64 einen ersten Bildaufnehmer 26-1 und einen zweiten Bildaufnehmer 26-2 auf. Demgemäß existieren zwei Aufnahmestrahlengänge. Einem ersten Aufnahmestrahlengang ist eine erste Aufnahmeoptik 66-1 zugeordnet. Einem zweiten Aufnahmestrahlengang ist eine zweite Aufnahmeoptik 66-2 zugeordnet. Ferner ist in Fig. 3 eine mit 68 bezeichnete Beleuchtungsoptik angeordnet, die der Beleuchtungseinheit 32 zugeordnet ist.

In einer beispielhaften Ausgestaltung ist die Schnittstelle 44 (Fig. 1) des optischen Instruments 12 ferner dazu ausgebildet, einen Lichtleiter bzw. eine externe Lichtquelle anzukoppeln. Demgemäß muss die Beleuchtungseinheit 32 (vgl. Fig. 1) nicht als aktive Beleuchtung gestaltet sein. Vielmehr kann die Beleuchtungseinheit 32 Lichtleiter umfassen, die den Schaft 40 zwischen dem proximalen Ende und dem distalen Ende durchlaufen und die in die Beleuchtungsoptik 68 münden.

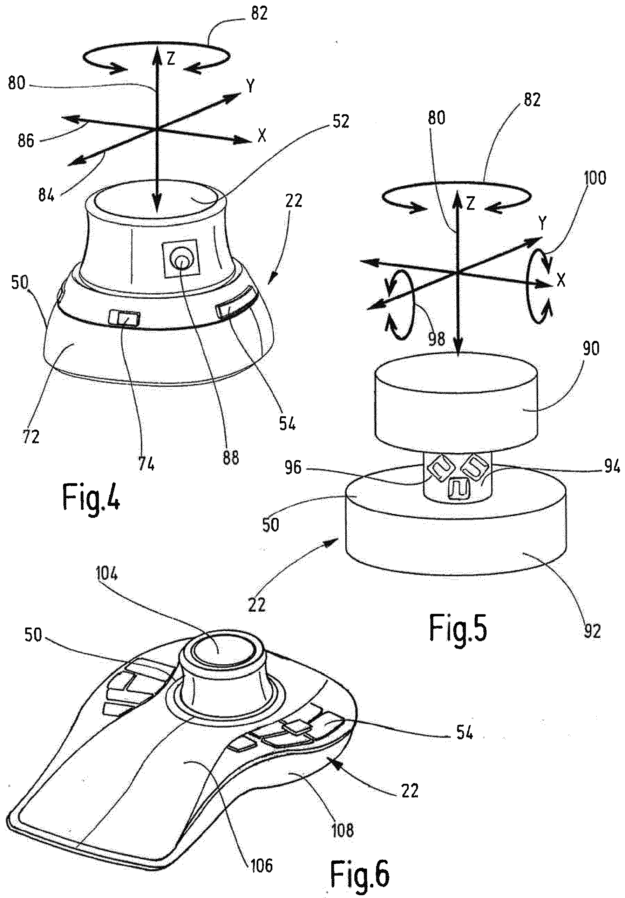

Verschiedene exemplarische Ausgestaltungen von Eingabemodulen 50, die bei der Bedieneinheit 22 verwendbar sind, werden nachfolgend anhand der Fig. 4, 5 und 6 näher veranschaulicht. Es versteht sich, dass Einzelaspekte eines der in den Fig. 4, 5 und 6 gezeigten Eingabemodule 50 auch auf die anderen Eingabemodule übertragbar sind.

Eine erste Ausgestaltung eines zur Einhandbedienung ausgestalteten Eingabemoduls 50 ist in Fig. 4 gezeigt. Das Eingabemodul 50 umfasst ein Hauptbetätigungselement 52, das etwa puckartig gestaltet ist. Das Betätigungselement 52 ist an einer Basis 72 des Eingabemoduls 50 aufgenommen. Ferner sind beispielhaft weitere (sekundäre) Betätigungselemente 54 vorgesehen, etwa Schalter, Taster oder dgl. Schließlich ist in Fig. 4 mit 74 eine Kommunikationsschnittstelle bezeichnet, über die Steuersignale ausgegeben werden können, die durch Betätigungen der Betätigungselemente 52, 54 erzeugbar sind.

Vorzugsweise sind die im Rahmen dieser Offenbarung vorgestellten Eingabemodule 50 als Mehrachs-Eingabemodule gestaltet, weiter bevorzugt als Vier-Achs-Eingabemodule. Eine beispielhafte Zuordnung von Bewegungsachsen bzw. Betätigungsachsen zum Eingabemodul 50 wird in Fig. 4 veranschaulicht. Fig. 5 zeigt eine alternative Zuordnung.

In Fig. 4 ist eine Translationsrichtung durch einen mit 80 bezeichneten Doppelpfeil veranschaulicht. Eine Rotationsrichtung 82, die eine Drehbewegung um die Achse der Translationsrichtung 80 beschreibt, ist durch einen mit 82 bezeichneten gekrümmten Doppelpfeil angedeutet. Demgemäß kann das Betätigungselement 52 translatorisch betätigt werden (Ziehen und Drücken). Daneben ist auch eine rotatorische Betätigung möglich (Drehen).

Weitere Betätigungsrichtungen sind in Fig. 4 mit 84 und 86 bezeichnet, die jeweilige Doppelpfeile kennzeichnen. Die Betätigungsrichtungen 84, 86 können auch als Schubrichtungen bezeichnet werden. Die Schubrichtungen 84, 86 spannen eine Ebene auf, in der das Betätigungselement 52 senkrecht zur Translationsrichtung 80 verschiebbar ist. Insbesondere kann das Betätigungselement 52 zumindest teilweise seitlich ausgelenkt werden, um ein Schubsignal zu erzeugen.

Beispielhaft sind die Achsen eines Koordinatensystems, das durch die Betätigungsrichtungen 80, 84, 86 definiert ist, mit X (vgl. 86), Y (vgl. 84) und Z (vgl. 80) bezeichnet. Es versteht sich, dass die genannte Zuordnung vorrangig Veranschaulichungszwecken dient. Abwandlungen sind ohne weiteres denkbar. Nötige gedankliche Transformationen kann der Fachmann ohne weiteres vornehmen. Um die Bewegungen unter Nutzung der Freiheitsgrade bzw. der Bewegungsrichtungen 80, 82, 84, 86 zu ermöglichen, ist das Betätigungselement 52 zumindest in Grenzen relativ zur Basis 72 bewegbar an dieser aufgenommen. So können etwa optische Sensoren vorgesehen sein, um die Auslenkungen zu erfassen und den jeweiligen Achsen zuzuordnen.

Vorzugsweise sind die im Rahmen dieser Offenbarung vorgestellten Eingabemodule 50 als Eingabemodule mit haptischer Rückmeldung ausgestaltet. Auf diese Weise kann einem Bediener, der das Eingabemodul 50 betätigt, eine Rückmeldung gegeben werden. Beispielhaft wird die haptische Rückmeldung durch einen Vibrationsmotor oder Vibrationserzeuger 88 generiert, der dem Eingabemodul 50, insbesondere dem Betätigungselement 52, zugeordnet ist. Die haptische Rückmeldung kann zum Beispiel das Erreichen von Grenzwerten oder Extremwerten signalisieren. Auf diese Weise kann einem Bediener signalisiert werden, dass bestimmte Bereiche oder Grenzwerte nicht überschritten werden dürfen.

Es ist auch vorstellbar, eine Mehrzahl von Vibrationserzeugern 88 vorzusehen, die zumindest einigen der Betätigungsrichtungen bzw. -achsen 80, 82, 84, 86 zugeordnet sind. Auf diese Weise kann eine noch feinere, dezidierte Rückmeldung erfolgen.

Fig. 5 veranschaulicht eine alternative beispielhafte Ausgestaltung eines Eingabemoduls 50 für eine Bedieneinheit 22, die bei der Beobachtungsvorrichtung 10 gemäß Fig. 1 verwendbar ist. Das Eingabemodul 50 umfasst ein scheibenartiges oder zylindrisches Betätigungselement 90, das grundsätzlich ähnlich dem Betätigungselement 52 gestaltet sein kann. Das Betätigungselement 90 ist an einer Basis 92 aufgenommen und über einen Schaft 94 mit der Basis 92 verbunden. Im Hinblick auf die Bewegungsachsen bzw. die Betätigungsachsen ist das anhand der Fig. 5 veranschaulichte Eingabemodul 50 gegenüber der anhand der Fig. 4 veranschaulichten Ausgestaltung abgewandelt. Wie bereits in Fig. 4 gezeigt, ist eine Translationsrichtung 80 und eine Rotationsrichtung 82 vorgesehen, wobei die Rotationsrichtung 82 Drehbewegungen um die Translationsrichtung 80 beschreibt. Anstatt der Schubrichtungen 84, 86 (vgl. Fig. 4) umfasst die Ausgestaltung gemäß Fig. 5 Schwenkrichtungen 98, 100, die eine jeweilige Verbiegung bzw. Verschwenkung des Betätigungselements 90 um die X-Achse bzw. die Y-Achse beschreiben. Auch über eine derartige Betätigungsbewegung kann ein entsprechendes Signal, das etwa eine Verschiebung in einem zweidimensionalen Raum beschreibt, erzeugt werden.

Das Betätigungselement 90 gemäß Fig. 5 ist starr an der Basis 92 aufgenommen. Mit anderen Worten ist das Betätigungselement 90 nicht bewegbar an der Basis 92 gelagert. Jedoch ist das Betätigungselement 90 abschnittsweise auslenkbar bzw. beweglich, da insbesondere der Schaft 94 nicht unendlich steif gestaltet ist. Auf diese Weise können definierte Verformungen am Schaft 94 erzeugt werden. Bei den Verformungen kann es sich etwa um Stauchungen, Längungen, Verbiegungen oder Tordierungen handeln. Derartige Verformungen können über Sensoren 96 erfasst werden, vorzugsweise über eine Mehrzahl derartiger Sensoren. Beispielhaft können die Sensoren 96 als Dehnmessstreifen ausgestaltet sein. Somit können auch minimale Verformungen am Schaft 94 erfasst und den Betätigungsrichtungen 80, 82, 98, 100 zugeordnet werden. Auch das Betätigungselement 90 gemäß Fig. 5 kann mit einem geeigneten Vibrationserzeuger 88 gekoppelt sein, um eine haptische Rückmeldung ausgeben zu können.

Die anhand der Fig. 5 veranschaulichte Ausführungsform des Eingabemoduls 50 ist integral gestaltet, da das Betätigungselement 90 fest mit der Basis 92 verbunden ist. Auf diese Weise ist das Eingabemodul 50 besonders robust gestaltet. Dies kann Reinigungsvorgänge, insbesondere Desinfektionsvorgänge oder Sterilisierungsvorgänge vereinfachen. Somit kann das Eingabemodul 50 in einfacher Weise in einem Autoklaven sterilisiert werden.

Fig. 6 veranschaulicht eine weitere Ausgestaltung eines zur Einhandbetätigung ausgestalteten Eingabemoduls 50, das bei der Bedieneinheit 22 zur Verwendung kommen kann. Das Eingabemodul 50 weist ein Betätigungselement 104 auf, das dem bereits anhand der Fig. 4 veranschaulichten Betätigungselement grundsätzlich ähnlich gestaltet ist. Ferner umfasst das Eingabemodul 50 eine Basis 108, an der eine Handauflage 106 ausgebildet ist. An der Basis 108 ist ferner eine Mehrzahl weiterer Betätigungselemente 54, insbesondere Taster, Schalter oder dgl., vorgesehen.

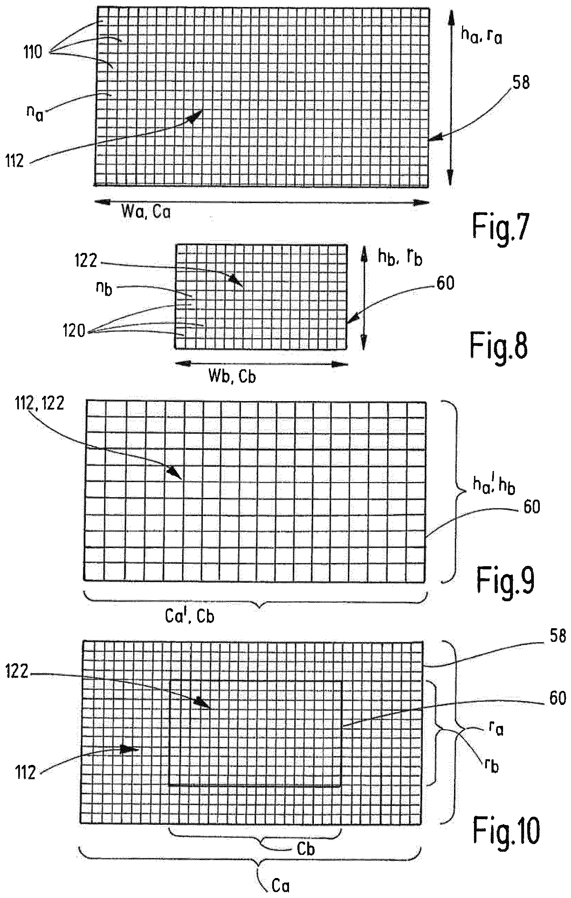

Mit Verweis auf die Fig. 7 und die Fig. 8 werden grundlegende Funktionen der Bildaufnahmeeinheit 18 sowie der Wiedergabeeinheit 16 näher erläutert. Fig. 7 zeigt ein Aufnahmebild 58, das durch die Bildaufnahmeeinheit 18 erfassbar und bereitstellbar ist. Fig. 8 zeigt ein Wiedergabebild 60, das durch die Wiedergabeeinheit 16 darstellbar ist. Nachfolgend wird davon ausgegangen, dass die Aufnahmebilder 58 sowie die Wiedergabebilder 60 einem Bildkanal einer stereoskopischen Darstellung, die zwei Bildkanäle umfasst, oder einem (einzigen) Bildkanal einer nicht-stereoskopischen Darstellung entsprechen. Eine Erweiterung auf stereoskopische Daten ist ohne weiteres vorstellbar.

Das Aufnahmebild 58 gemäß Fig. 7 weist eine Vielzahl von Pixeln 110 auf. Eine Pixelmenge des Aufnahmebilds 58 wird mit nₐ bezeichnet. Abmessungen des Aufnahmebilds 58 sind in Fig. 7 mit wₐ und hₐ bezeichnet. Die Breite des Aufnahmebilds 58 ist mit wₐ bezeichnet und umfasst eine definierte Anzahl von Pixelspalten cₐ. Die Höhe des Aufnahmebilds 58 ist mit hₐ bezeichnet und umfasst eine definierte Anzahl von Pixelreihen ra.

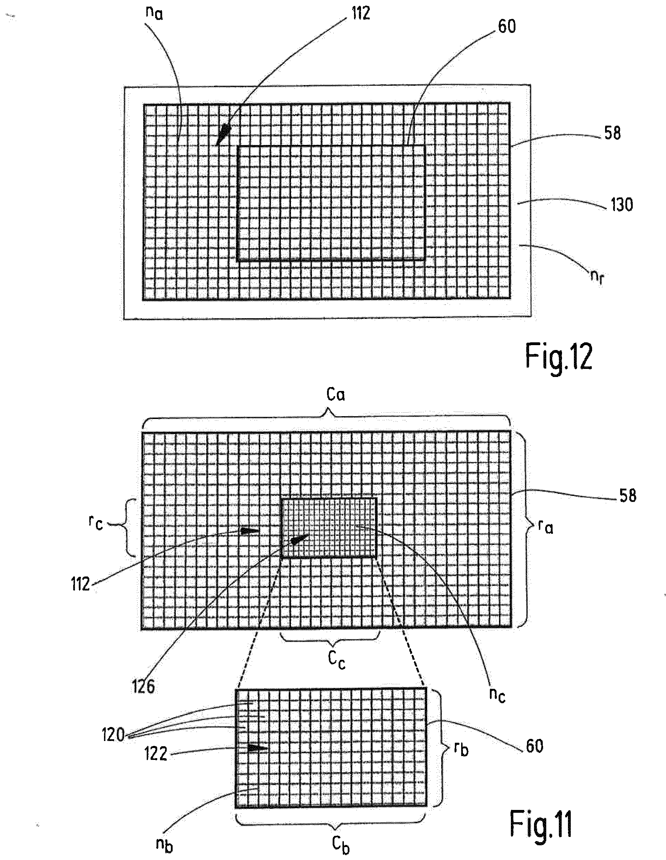

Das Aufnahmebild 58 deckt einen Aufnahmebereich 112 ab. Der Aufnahmebereich 112 entspricht etwa dem Bereich der Objektebene 28 (vgl. Fig. 1), der grundsätzlich mit dem Bildaufnehmer 26 erfassbar ist (vgl. jedoch die weiteren Ausführungen in Zusammenhang mit Fig. 12).

Das in Fig. 8 gezeigte Wiedergabebild 60 weist eine Vielzahl von Pixeln 120 auf. Eine Pixelmenge des Wiedergabebildes 60 beträgt n_{b}. Abmessungen des Wiedergabebildes 60 sind in Fig. 8 mit w_{b} und h_{b} bezeichnet, wobei eine Breite mit w_{b} und eine Höhe mit h_{b} bezeichnet ist. Die Breite w_{b} umfasst eine definierte Anzahl von Pixelspalten c_{b}. Die Höhe h_{b} umfasst eine definierte Anzahl von Pixelreihen r_{b}. Die Pixel 120 des Wiedergabebildes 60 definieren einen Wiedergabebereich 122.

Die Pixelmengen nₐ und n_{b} sind vorzugsweise derart definiert, dass die Pixelmenge nₐ des Aufnahmebereichs 112 ein ganzzahliges Vielfaches der Pixelmenge n_{b} des Wiedergabebereichs 122 ist. Beispielhaft ist der Wiedergabebereich 122 etwa als HD-Bereich (1280 × 720 Pixel) oder als Full HD-Bereich (1920 × 1080 Pixel) ausgeführt. Demgemäß kann der Aufnahmebereich 112 beispielhaft etwa das Zweifache, Dreifache, Vierfache, Sechsfache oder gar Achtfache der Pixel des Wiedergabebereichs 122 umfassen.

Das Seitenverhältnis (cₐ:rₐ) des Aufnahmebereichs 112 entspricht vorzugsweise dem Seitenverhältnis (c_{b}:r_{b}) des Wiedergabebereichs 122.

Die zur Darstellung der Aufnahmebilder 58 bzw. der Wiedergabebilder 60 genutzte karierte Darstellung bzw. Kästchendarstellung veranschaulicht entsprechende Pixelmengen nₐ bzw n_{b}. Ein Vergleich der Fig. 7 mit der Fig. 8 zeigt demgemäß, dass der durch das Wiedergabebild 60 darstellbare Wiedergabebereich 122 nur einem Teilbereich des Aufnahmebereichs 112 entspricht, wenn die (Detail-)Auflösung oder Pixeldichte beibehalten wird, wenn also ein zusammenhängender Bereich der Pixel 110 des Aufnahmebereichs 112 im Wiedergabebereich 122 dargestellt werden soll. Demgemäß sind die Abmessungen w_{b}, h_{b} des Wiedergabebereichs 122, bezogen auf die Objektebene 28, kleiner als die Abmessungen wₐ, hₐ des potenziell verfügbaren Aufnahmebereichs 112.

Fig. 9 veranschaulicht einen Wiedergabemodus, bei dem das Wiedergabebild 60 den gesamten Aufnahmebereich 112 des Aufnahmebilds 58 wiedergibt. Demgemäß entspricht der Wiedergabebereich 122 grundsätzlich dem Aufnahmebereich 112. Jedoch ist die Pixeldichte deutlich geringer, da im Wiedergabebereich 122 nur eine begrenzte Anzahl an Pixeln 120 zur Verfügung steht. Mit anderen Worten wird etwa nur jedes zweite oder nur jedes vierte der Pixel 110 des Aufnahmebereichs 112 für die Wiedergabe im Wiedergabebereich 122 genutzt. Es versteht sich, dass auch ein entsprechender Mittelwert benachbarter Pixel nutzbar ist, um die Pixelmenge nₐ des Aufnahmebereichs 112 in die Pixelmenge n_{b} des Wiedergabebereichs 122 zu überführen. Demgemäß ist die Darstellung in Fig. 9 grober gerastert als die Darstellung in Fig. 7. In Fig. 9 stellen cₐ' und hₐ' eine von den Ausgangswerten cₐ und hₐ abgeleitete Spaltenanzahl bzw. Zeilenanzahl dar, die hier jeweils den Werten von c_{b} und h_{b} entsprechen.

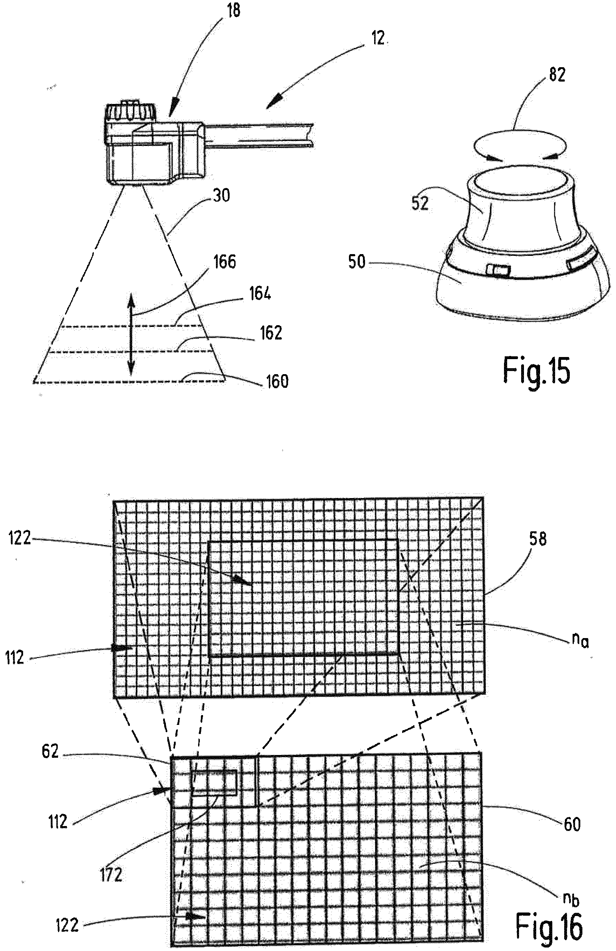

Fig. 10 zeigt einen Darstellungsmodus, bei dem das Wiedergabebild 60 zur Wiedergabe lediglich eines Abschnitts des Aufnahmebildes 58 genutzt wird. Im Vergleich zur Fig. 9 zeigt das Wiedergabebild 60 in Fig. 10 nicht den gesamten Aufnahmebereich 112. Mit anderen Worten ist die Abmessung des Wiedergabebereichs 122 c_{b}, r_{b} kleiner als die Abmessung cₐ, rₐ des Aufnahmebereichs 112. Im Vergleich zur Darstellung gemäß Fig. 9 werden bei der Darstellung gemäß Fig. 10 mehr Pixel oder alle Pixel im gewählten Ausschnitt des Aufnahmebereichs 112 im Wiedergabebereich 122 dargestellt. Je nach gewählter Vergrößerung kann etwa eine 1:1-Wiedergabe zumindest einer Teilmenge der Pixel 110 des Aufnahmebereichs 112 im Wiedergabebereich 122 erfolgen. Fig. 9 zeigt eine Übersicht ohne Vergrößerung. Fig. 10 zeigt eine mittlere Vergrößerungsstufe, bei der ein Ausschnitt des Aufnahmebildes 58 im Wiedergabebild 60 wiedergegeben wird, wobei der Ausschnitt in dieser Vergrößerungsstufe derart gewählt ist, dass eine zusammenhängende Teilmenge der Pixel 110 des Aufnahmebereichs 112 die Pixel 120 des Wiedergabebereichs 122 bildet, vergleiche hierzu auch die Darstellung in Fig. 16, bei der das Aufnahmebilde 58 und das Wiedergabebild 60 zur besseren Veranschaulichung separat dargestellt werden.

Fig. 11 veranschaulicht eine weitere Vergrößerungsstufe, bei der ein noch kleinerer Ausschnitt 126 des Aufnahmebereichs 112 im Wiedergabebereich 122 dargestellt wird. Der Ausschnitt 126 erstreckt sich im Aufnahmebereich 112 über eine definierte Anzahl von Pixeln n_{c}, die durch eine definierte Anzahl von Pixelspalten c_{c} und eine definierte Anzahl von Pixelreihen r_{c} definiert ist. Die Pixelmenge n_{c} des Ausschnitts 126 ist kleiner als die Pixelmenge n_{b}, die im Wiedergabebereich 122 darstellbar ist (vgl. die Darstellung des Ausschnitts 126 in Fig. 11, wobei zur Veranschaulichung die größere (Wiedergabe-)Pixelmenge n_{b} der kleineren (Sensor-)Pixelmenge n_{c} überlagert ist). Mit anderen Worten können mehrere Wiedergabepixel einem Aufnahmepixel entsprechen. Demgemäß erfolgt eine Interpolation bzw. rechnerische "Hochskalierung", um auf Basis der begrenzten Pixelmenge n_{c} die Pixel 120 (Pixelmenge n_{b}) des Wiedergabebildes 60 abzuleiten.

Auf diese Weise kann eine höhere Vergrößerung bewirkt werden als z.B. im Vergrößerungsmodus gemäß Fig. 10, wobei die Vergrößerung nicht mehr verlustfrei erfolgt. Fig. 10 zeigt eine verlustfreie Vergrößerung. Je nach dem Verhältnis zwischen der Anzahl der Pixel nₐ des Aufnahmebereichs 112 und der Anzahl der Pixel n_{b} des Wiedergabebereichs 122 können zumindest zwei verlustfreie Wiedergabestufen bzw. Abbildungsmaßstäbe (vgl. Übersichtsdarstellung in Fig. 9 und Vergrößerung in Fig. 10) oder gar drei, vier oder mehr verlustfreie Abbildungsmaßstäbe bereitgestellt werden.

Es versteht sich, dass auch geeignete Zwischenstufen darstellbar sind, sofern dies gewünscht ist. Entsprechende Interpolationen sind denkbar. Sofern eine Darstellung gewünscht ist, die eine über die in Fig. 10 gezeigte Vergrößerung (1:1 -Darstellung) hinausgeht, muss auf Interpolationsverfahren zurückgegriffen werden. Jedoch hat sich gezeigt, dass die sich ergebenden Wiedergabebilder 60 hinreichend detailliert sind, wenn die definierte Pixelmenge n_{b}, von der für den Wiedergabebereich ausgegangen wird, hinreichend groß ist, etwa Full HD. Eine 1:1 Darstellung (eins-zu-eins Darstellung) kommt dann zustande, wenn die Pixelmenge in gewähltem Ausschnitt 122 im Aufnahmebereich 112 genau (oder zumindest im Wesentlichen) der Pixelmenge im Wiedergabebereich 122 entspricht.

Fig. 12 veranschaulicht eine weitere beispielhafte Ausgestaltung, die insbesondere die Bildaufnahmeeinheit 18 bzw. den zumindest einen Bildaufnehmer 26 betrifft. Fig. 12 veranschaulicht beispielhaft ein Aufnahmebild 58 sowie ein gewähltes Wiedergabebild 60 im Aufnahmebild 58. Der zumindest eine Bildaufnehmer 26 kann grundsätzlich dazu ausgestaltet sein, Bilddaten in einem Gesamterfassungsbereich 130 zu erfassen, der größer als der Aufnahmebereich 112 des Aufnahmebildes 58 ist. Insbesondere kann sich ein Rahmen ergeben, der den Aufnahmebereich 112 überlappt. Beispielhaft umfasst der Gesamterfassungsbereich 130 eine Rohdatenpixelmenge nᵣ, die größer als die Pixelmenge nₐ des Aufnahmebereichs 112 ist.

Der Aufnahmebereich 112 kann etwa derart im Gesamterfassungsbereich 130 gewählt werden, dass ein ganzzahliges Verhältnis zwischen der Pixelmenge nₐ des Aufnahmebereichs 112 und der Pixelmenge n_{b} des Wiedergabebereichs 122 gewahrt ist. Ein weiterer Vorteil kann darin bestehen, den Aufnahmebereich 112 bewusst kleiner als den Gesamterfassungsbereich 130 zu wählen, um störende Effekte zu vermeiden, die häufig in Randbereichen von Bildaufnehmern 26 auftreten können.

Ein weiterer Vorteil der anhand der Fig. 12 veranschaulichten Ausgestaltung kann sich etwa im Hinblick auf die stereoskopische Bildverarbeitung und Bilddarstellung ergeben. Der Ausschnitt des Gesamterfassungsbereichs 130, der den Aufnahmebereich 112 des Bildaufnehmers 26 bildet, kann zumindest in engen Grenzen verschoben werden. Dies kann bei der Justage bzw. Feineinstellung der Bildaufnahmeeinheit 18 von Vorteil sein. Ferner kann, bei Verwendung zweier Bildaufnehmer 26, die zwei Halbbilder für die stereoskopische Darstellung erfassen, ein gewünschter Bildabstand zwischen den Halbbildern durch Verschieben der Aufnahmebereiche 112 in den Gesamterfassungsbereichen 130 eingestellt werden. Auf diese Weise kann die Justage und Feineinstellung softwaremäßig erfolgen. Idealerweise kann auf aufwendige mechanische oder optische Justagearbeiten verzichtet werden.

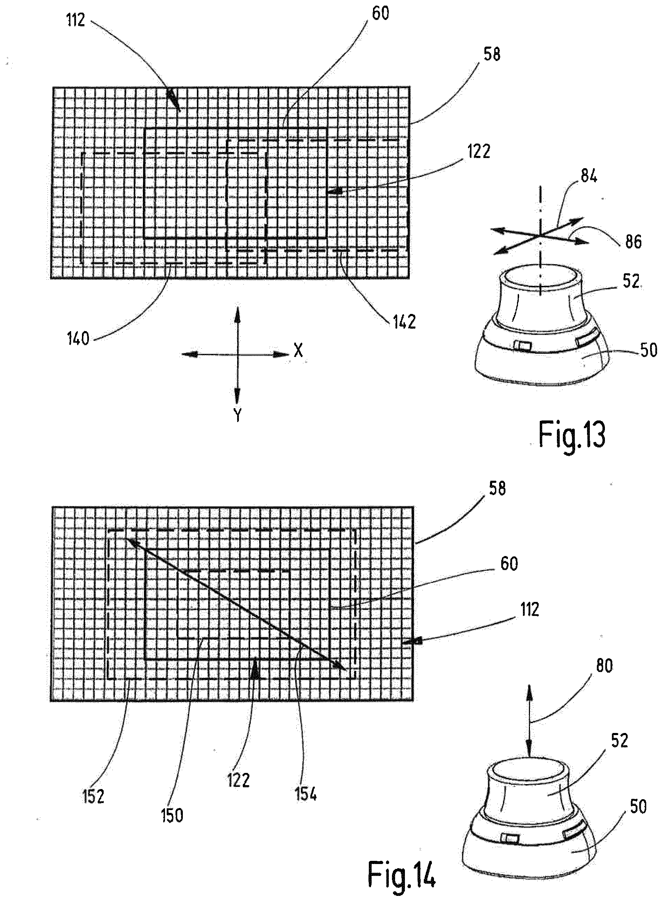

Anhand der Fig. 13, 14 und 15 wird eine vorteilhafte Zuordnung von Betätigungsachsen des Eingabemoduls 50 zur Steuerung von Bildaufnahmeparametern bzw. Wiedergabeparametern veranschaulicht.

Fig. 13 zeigt ein Wiedergabebild 60, das einen Wiedergabebereich 122 umfasst, der als Teilmenge des Aufnahmebereichs 112 des Aufnahmebildes 58 gewählt ist. Der Wiedergabebereich 122 kann innerhalb der Grenzen, die durch den Aufnahmebereich 112 definiert sind, verschoben werden, vgl. mit X und Y bezeichnete Pfeile in Fig. 13. Diese Schubbewegung kann etwa über in den Schubrichtungen 84, 86 des Eingabemoduls 50 bewirkt werden. Eine entsprechende Verschiebung ist jedoch auch über die Schwenkrichtungen 98, 100 steuerbar, vgl. hierzu auch Fig. 5. Verschobene Wiedergabebereiche sind in Fig. 13 mit 140, 142 bezeichnet.

Fig. 14 veranschaulicht eine Zoom-Funktion, bei der unterschiedlich große Bereiche des Aufnahmebildes 58 ausgewählt werden, um den Wiedergabebereich 122 zu definieren, auf dessen Basis das Wiedergabebild 60 abgeleitet wird.

Ein kleinerer Ausschnitt ist mit 150 bezeichnet. Der kleinere Ausschnitt bewirkt im entstehenden Wiedergabebild eine Vergrößerung. Ein vergrößerter Ausschnitt ist mit 152 bezeichnet. Der Ausschnitt 152 bewirkt bei der Wiedergabe im Wiedergabebild eine Verkleinerung. Ein mit 154 bezeichneter Doppelpfeil veranschaulicht die Skalierung des Wiedergabebereichs 122 zur Erzeugung verschiedener Vergrößerungsstufen.

Die Skalierung kann durch eine Betätigung des Eingabemoduls 50 in der Translationsrichtung 80 bewirkt werden, also etwa durch ein Drücken oder Ziehen des Betätigungselements 52.

Fig. 15 veranschaulicht eine Fokusverstellung, die grundsätzlich zusätzlich zur Verschiebung gemäß Fig. 13 und zusätzlich zur Größenverstellung gemäß Fig. 14 mit dem Eingabemodul 50 bewirkt werden kann. Beispielhaft umfasst die Bildaufnahmeeinheit 18 einen Fokusantrieb bzw. eine Fokusverstellung. Das Sichtfeld 30 bzw. der konzeptbedingte Öffnungswinkel des Sichtfelds 30 ist jedenfalls bei denjenigen Ausgestaltungen, die keinen optischen Zoom umfassen, nicht ohne weiteres variierbar. Jedoch kann der Abstand einer Fokusebene (auch: Schärfeebene) 160, 162, 164 mit hinreichend scharfer Darstellung von der Bildaufnahmeeinheit 18 variiert werden, um Objekte mit unterschiedlichem Abstand zur Bildaufnahmeeinheit 18 scharf zu stellen. Mit anderen Worten kann etwa wahlweise ein Objekt, das sich im Vordergrund befindet, oder ein Objekt, das sich im Hintergrund befindet, scharf gestellt werden. Eine entsprechende Verschiebung der Schärfeebene ist in Fig. 15 durch einen mit 166 bezeichneten Doppelpfeil angedeutet. Hierfür werden über den Fokusantrieb Fokuslinsen im optischen Instrument verschoben. Gesteuert wird die Verschiebung über das Eingabemodul 50.

Die Schärfenverstellung kann etwa durch Betätigung in der Rotationsrichtung 82 vorgenommen werden. Zu diesem Zweck kann das Betätigungselement 52 um seine Längsachse verdreht werden.

Es versteht sich, dass auch alternative Gestaltungen denkbar sind, bei denen etwa die Größenverstellung durch Drehen des Betätigungselements 52 und die Verstellung der Schärfeebene durch Ziehen bzw. Drücken des Betätigungselements 52 bewirkt werden kann.

Die anhand der Fig. 13, 14 und 15 veranschaulichten Funktionen umfassen allesamt definierte Verstellbereiche. Sofern Grenzwerte bzw. Extremzustände erreicht werden, kann am Eingabemodul 50 eine haptische Rückmeldung ausgegeben werden, um dies dem Bediener zu signalisieren. Dies kann etwa bei der Verschiebung des Ausschnitts gemäß Fig. 13 das Erreichen des Rands des Aufnahmebereichs 112 signalisieren. Bei der in Fig. 14 gezeigten Funktion kann beispielsweise durch eine haptische Rückmeldung signalisiert werden, dass der gewählte Wiedergabebereich 122 dem Aufnahmebereich 112 entspricht und folglich keine kleinere Zoom-Stufe ermöglicht ist. Umgekehrt kann signalisiert werden, dass eine extreme Vergrößerung (extrem kleiner Wiedergabebereich 122) gewählt ist, und dass eine weitere Vergrößerung nicht möglich ist.

In ähnlicher Weise kann bei der Schärfeebenenverstellung gemäß Fig. 15 eine Rückmeldung ausgegeben werden, wenn ein minimaler Abstand oder ein maximaler Abstand erreicht ist.

Fig. 16 veranschaulicht eine weitere beispielhafte Funktion, die die Beobachtungsvorrichtung bereitstellen kann. In bereits weiter oben beschriebener Weise zeigt Fig. 16 beispielhaft ein Aufnahmebild 58 sowie ein Wiedergabebild 60, das auf Basis eines Ausschnitts des Aufnahmebildes 58 erzeugt ist, der einen Wiedergabebereich 122 definiert. Das Wiedergabebild 60 deckt den Wiedergabebereich 122 ab. Gemäß zumindest einiger Ausgestaltungen ist es vorstellbar, zumindest zeitweise ein Übersichtsbild 62 im Wiedergabebild 60 anzuzeigen, das den Wiedergabebereich 122 zumindest teilweise überlagert. Dies kann etwa als Bild-in-Bild-Darstellung erfolgen. Vorzugsweise deckt das Übersichtsbild 62 den gesamten Aufnahmebereich 112 ab, wenn auch in einer grob aufgelösten Darstellung. Auf diese Weise kann dem Bediener eine optische Orientierungshilfe bereitgestellt werden. Vorzugsweise wird ferner im Übersichtsbild 62 ein Ausschnittsbereich 172 dargestellt, der als Positionsindikator dient. Der Ausschnittsbereich 172 kennzeichnet die Position des gewählten Wiedergabebereichs 122 im Aufnahmebereich 112 des Aufnahmebildes 58.

Es ist vorstellbar, das Übersichtsbild 62 zumindest dann einzublenden, wenn eine der in den Fig. 13, 14 oder 15 gezeigten Funktionen genutzt wird. Bei Bedarf kann der Übersichtsbereich 62 etwa auch auf Knopfdruck angezeigt und ausgeblendet werden. Der Übersichtsbereich 62 kann zumindest teilweise transparent gestaltet werden, um einen darunterliegenden Bereich des Wiedergabebereichs 122 zumindest teilweise sichtbar zu machen.

Die anhand der Fig. 13, 14 und 15 veranschaulichten Funktionen zeigen, dass das Eingabemodul 50 dazu nutzbar ist, Bildaufnahmeparameter und Wiedergabeparameter zu steuern. Dies kann gleichzeitig erfolgen. Ein Bildaufnahmeparameter ist beispielsweise die aktuelle Lage der Schärfeebene. Ein Wiedergabeparameter ist beispielsweise eine aktuell gewählte Vergrößerungsstufe oder eine aktuelle Position des gewählten Bildausschnitts (Wiedergabebereichs) im grundsätzlich zur Verfügung stehenden Aufnahmebereich.

Es versteht sich, dass das Aufnahmebild 58, das in verschiedenen der hier gezeigten Figuren dargestellt ist, nicht sichtbar zur Verfügung stehen muss. Vielmehr kann das Aufnahmebild 58 in Form einer entsprechenden Datenverkörperung vorliegen. Beispielhaft ist es vorstellbar, dass der Aufnahmebereich konstant, mit einer gewählten Wiederholrate, ausgelesen wird und potenziell zur Verfügung steht. Somit kann die Bildaufnahmeeinheit 18 einen Aufnahmedatenstrom bereitstellen, welcher vollständig oder teilweise nutzbar ist, um gewünschte Wiedergabebilder abzuleiten.

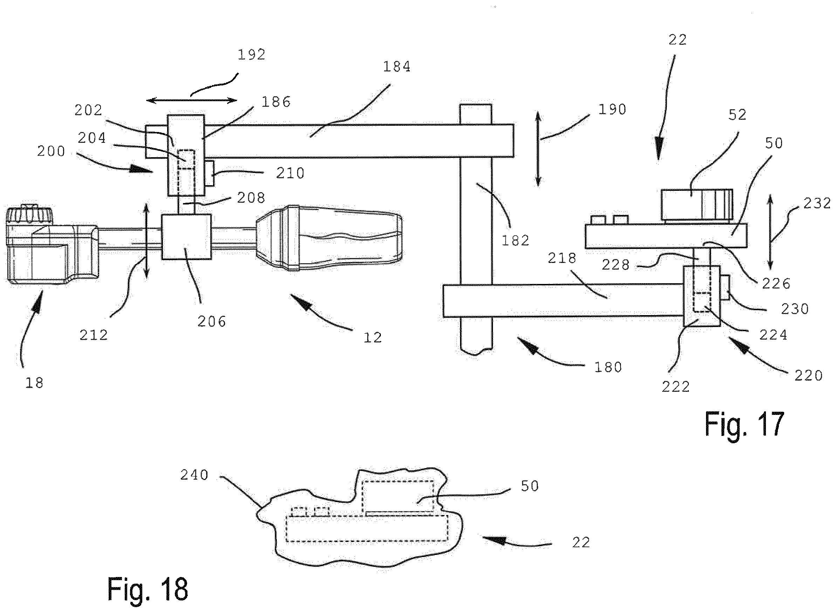

Fig. 17 zeigt eine schematische seitliche Ansicht eines weiteren Ausführungsbeispiels einer Beobachtungsvorrichtung 10. In Fig. 17 ist eine gelenkige Halterung 180 dargestellt. Die Halterung 180 weist beispielweise einen Arm 182 auf, an dem ein weiterer Arm 184 aufgenommen ist. Die Arme 182, 184 können auch als Säulen oder Gelenke bezeichnet werden. Die Halterung 180 ist in Fig. 17 lediglich teilweise gezeigt. Die Halterung 180 kann auch als Stativ oder Gestell gestaltet sein.

Ein Schlitten 186 ist beweglich am Arm 184 aufgenommen. Der Arm 184 ist beweglich am Arm 182 aufgenommen. Folglich kann die Halterung 180 mit einem Positionierantrieb 190, 192 versehen sein, wobei die Bezugszeichen 190, 192 in Fig. 17 Bewegungsrichtungen zwischen den Armen 182, 184 bzw. zwischen dem Arm 184 und dem Schlitten 186 bezeichnen. Beim Schlitten 186 ist ein optisches Instrument 12 mit einer Bildaufnahmeeinheit 18 lösbar an der Halterung 180 aufgenommen. Folglich kann der Positionierantrieb 190, 192 dazu genutzt werden, um die Bildaufnahmeeinheit 18 zu bewegen.

In beispielhaften Ausgestaltungen wird der Positionierantrieb auch über die Bedieneinheit 22 gesteuert, insbesondere über das Eingabemodul 50 der Bedieneinheit 22. Auf diese Weise kann eine Verstellung oder Anpassung eines Erfassungsbereichs der Bildaufnahmeeinheit 18 bewirkt werden. Da das Eingabemodul 50 zumindest gemäß beispielhaften Ausgestaltungen als Mehrachs-Bedienmodul gestaltet ist, können verschiedenen Achsen des Positionierantriebs gleichzeitig gesteuert werden. Um das optische Instrument 12 lösbar und genau an der Halterung 180 aufzunehmen, ist eine Montageschnittstelle 200 vorgesehen. Gemäß beispielhaften Ausgestaltungen umfasst die Montageschnittstelle 200 eine Schnelllösekupplung (Quick lock Kupplung). Die Montageschnittstelle 200 umfasst einen Sockel 202, der mit einer Ausnehmung 204 versehen ist. Ferner ist ein Aufnahmehalter 206 vorgesehen, der einen schaftartigen Montagevorsprung 208 aufweist. Gemäß der beispielhaften Ausgestaltung, die in Fig. 17 gezeigt ist, ist der Sockel 202 dem Schlitten 186 zugeordnet. Ferner ist der Aufnahmehalter 206 als Halter für das optische Instrument 12 ausgebildet. Der Aufnahmehalter 206 kann als Halteklemme gestaltet sein. Vorzugsweise weit die Montageschnittstelle 200 eine Verriegelung auf, die über einen Verriegelungsbetätiger 210 betätigbar ist. Um das optische Instrument 12 an der Halterung 180 festzulegen, wird der Montagevorsprung 208 in die Ausnehmung 204 eingeführt und dort verriegelt, vergleiche auch eine entsprechende Richtung 212 der Montagebewegung in Fig. 17. Der Verriegelungsbetätiger 210 kann eine Verriegelungsmechanik steuern, zum Beispiel eine formschlüssige und/oder kraftschlüssige Verriegelungsmechanik. Der Verriegelungsbetätiger 210 ist dazu ausgestaltet, die Ausnehmung 204 und den Montagevorsprung 208 zu verriegeln oder zu entriegeln.

Gemäß der in Fig 17 gezeigten beispielhaften Ausgestaltung ist auch die Bedieneinheit 22, insbesondere das Eingabemodul 50 mit dem Betätigungselement 52 an der Halterung 180 aufgenommen. Beispielsweise ist die Bedieneinheit 22 an einem Arm 218 aufgenommen, der am Arm 182 aufgenommen ist. Alternativ kann die Bedieneinheit 22 ebenso an einer separaten Halterung bzw. einem separaten Stativ aufgenommen sein. In jedem Fall kann eine Montageschnittstelle 200 vorgesehen sein, um die Bedieneinheit 22 lösbar an der Halterung 180 aufzunehmen. Gemäß beispielhaften Ausgestaltungen umfasst die Montageschnittstelle 220 eine Schnelllösekupplung (Quick lock Kupplung). Die Montageschnittstelle 200 umfasst eine Aufnahmebasis 222, die mit einer Ausnehmung 224 versehen ist. Ferner ist ein Aufnahmehalter 206 vorgesehen, der einen schaftartigen Montagevorsprung 228 aufweist. Gemäß der beispielhaften Ausgestaltung, die in Fig. 17 gezeigt ist, ist die Aufnahmebasis 222 am Arm 218 aufgenommen. Die Aufnahmebasis 222 ist beim Eingabemodul 50 angeordnet oder als Teil davon ausgebildet. Vorzugsweise weit die Montageschnittstelle 220 eine Verriegelung auf, die über einen Verriegelungsbetätiger 230 betätigbar ist. Um die Bedieneinheit 22 an der Halterung 180 festzulegen, wird der Montagevorsprung 228 in die Ausnehmung 224 eingeführt und dort verriegelt, vergleiche auch eine entsprechende Richtung 232 der Montagebewegung in Fig. 17. Der Verriegelungsbetätiger 230 kann eine Verriegelungsmechanik steuern, zum Beispiel eine formschlüssige und/oder kraftschlüssige Verriegelungsmechanik. Der Verriegelungsbetätiger 230 ist dazu ausgestaltet, die Ausnehmung 224 und den Montagevorsprung 228 zu verriegeln oder zu entriegeln.

In Fig. 17 sind die Montageschnittstellen 200, 220 in einem teilweise gelösten Zustand dargestellt. Die Montageschnittstellen 200, 220 können die gleichen Passelemente nutzen (Ausnehmung, Vorsprung, Verriegelungsbetätiger), so dass eine Austauschbarkeit gegeben ist.

Fig. 18 ist eine schematische Seitenansicht einer Anordnung einer Bedieneinheit 22 mit einem Einhand-Eingabemodul 50, mit einer sterilen Hülle 240 bedeckt ist. Folglich ist selbst dann, wenn das Eingabemodul 50 nicht autoklavierbar oder sterilisierbar ist, eine Nutzung bei medizinischen Anwendungen möglich. Die sterile Hülle kann zur Einmalverwendung gestaltet sein.

## Patentansprüche

1. Beobachtungsvorrichtung (10), insbesondere medizinische Beobachtungsvorrichtung, die Folgendes aufweist:
- eine Bildaufnahmeeinheit (18) mit zumindest einem Bildaufnehmer (26), vorzugsweise eine Stereo-Bildaufnahmeeinheit mit zwei Bildaufnehmern,
- eine Wiedergabeeinheit (16), die zur Wiedergabe von Bilddaten ausgestaltet ist, die durch die Bildaufnahmeeinheit (18) bereitgestellt werden,
- eine Bildverarbeitungseinheit (20) für Bildverarbeitungsprozesse,
- eine Bedieneinheit (22) mit einem Mehrachs-Eingabemodul (50), und
- einen Positionierantrieb (190, 192) für die Bildaufnahmeeinheit (18),
wobei Bildaufnahmeparameter und Wiedergabeparameter über das Eingabemodul (50) steuerbar sind,
wobei das Eingabemodul (50) zumindest in einem ersten Betriebsmodus und einem zweiten Betriebsmodus betreibbar ist,
wobei im ersten Betriebsmodus eine direkte Steuerung von Bildaufnahmeparametern und Wiedergabeparametern ermöglicht ist,
wobei das Eingabemodul (50) im zweiten Betriebsmodus betätigbar ist, um den Positionierantrieb (190, 192) zur Positionierung der Bildaufnahmeeinheit (18) zu steuern, und
wobei das Eingabemodul (50) als Einhand-Eingabemodul ausgestaltet ist und ein durch einen Bediener manipulierbares Betätigungselement (52, 90, 104) aufweist,
das mehrere Bewegungsfreiheitsgrade (80, 82, 84, 86; 80, 82, 98, 100) für Eingaben bereitstellt,
**dadurch gekennzeichnet,**
**dass** verschiedene Achsen des Positionierantriebs (190, 192) gleichzeitig durch das Eingabemodul (50) steuerbar sind, und
**dass** die direkte Steuerung von Wiedergabeparametern eine Positionierung eines Wiedergabebildes (60) in einem bereitgestellten globalen Aufnahmebild (58) umfasst.

2. Beobachtungsvorrichtung (10) nach Anspruch 1, wobei die Bildaufnahmeeinheit (18) dazu ausgebildet ist, Aufnahmebilder (58) einer vordefinierten Aufnahmepixelmenge (nₐ) bereitzustellen, wobei die Wiedergabeeinheit (16) dazu ausgebildet ist, Wiedergabebilder (60) einer vordefinierten Wiedergabepixelmenge (n_{b}) darzustellen, wobei die Aufnahmepixelmenge (nₐ) größer oder gleich der Wiedergabepixelmenge (n_{b}) ist, wobei Bildpixel (120) der Wiedergabepixelmenge (n_{b}) der Aufnahmepixelmenge (nₐ) entnehmbar sind, wobei, zur Bereitstellung von Ansichten mit unterschiedlichen Vergrößerungen, Teilmengen der Aufnahmepixelmenge (nₐ) wählbar sind, um die Wiedergabepixelmenge (n_{b}) zu bilden, und wobei das Eingabemodul (50) zur Steuerung zumindest eines Bildaufnahmeparameters mit der Bildaufnahmeeinheit (18) koppelbar ist.

3. Beobachtungsvorrichtung (10) nach Anspruch 2, wobei das Eingabemodul (50) mit der Bildaufnahmeeinheit (18) und der Bildverarbeitungseinheit (20) gekoppelt ist, um zur Variation von Bildaufnahmeparametern auf die Bildaufnahmeeinheit (18) sowie zur Variation von Wiedergabeparametern auf die Bildverarbeitungseinheit (20) einzuwirken, und wobei im ersten Betriebsmodus eine direkte Steuerung von Bildaufnahmeparametern und Wiedergabeparametern mit dem Eingabemodul (50) ermöglicht ist, wobei im zweiten Betriebsmodus eine Steuerung weiterer peripherer Funktionen mit dem Eingabemodul (50) ermöglicht ist.

4. Beobachtungsvorrichtung (10) nach einem der vorhergehenden Ansprüche, wobei das Betätigungselement (52, 90, 104) puckartig oder knaufartig gestaltet ist, wobei das Betätigungselement (52, 90, 104) mit Sensoren (96) gekoppelt ist, um eine Zug-/Druckbewegung entlang einer Längsachse (Z) des Betätigungselements (52, 90, 104), eine Drehbewegung um die Längsachse (Z), und Schubbewegungen in einer Ebene (X, Y), die senkrecht zur Längsachse (Z) orientiert ist oder Schwenkbewegungen um Schwenkachsen (X, Y), die senkrecht zur Längsachse (Z) orientiert sind, zu erfassen.

5. Beobachtungsvorrichtung (10) nach einem der vorhergehenden Ansprüche, wobei das Betätigungselement (52, 90, 104) mit zumindest einem als Wegaufnehmer oder Kraftaufnehmer ausgebildeten Sensor (96) gekoppelt ist, vorzugsweise mit einer Mehrzahl von Sensoren zur multiaxialen Erfassung von Verformungen oder Bewegungen.

6. Beobachtungsvorrichtung (10) nach einem der vorhergehenden Ansprüche, wobei das Betätigungselement (52, 90, 104) als Vier-Achs-Betätigungselement gestaltet ist, wobei eine Betätigung der ersten Betätigungsachse (80) eine Vergrößerung und eine der Vergrößerung zugeordnete Größe eines Bereichs (122) der Wiedergabebilder (60) in den Aufnahmebildern (58) definiert, wobei eine Betätigung der zweiten Betätigungsachse (82) eine Fokuseinstellung definiert, wobei eine Betätigung der dritten Betätigungsachse (84) eine Bewegung des von den Wiedergabebildern (60) abgedeckten Bereichs (122) in einem durch die Aufnahmebilder (58) abgedeckten Bereich (112) in einer ersten Bewegungsrichtung (X) bewirkt, und wobei eine Betätigung der vierten Betätigungsachse (86) eine Bewegung des von den Wiedergabebildern (60) abgedeckten Bereichs (122) in dem durch die Aufnahmebilder (58) abgedeckten Bereich (112) in einer zweiten Bewegungsrichtung (Y) bewirkt, die zur ersten Bewegungsrichtung (X) geneigt ist.

7. Beobachtungsvorrichtung (10) nach Anspruch 6, wobei die erste Betätigungsachse eine Translationsrichtung (80) bereitstellt, wobei die zweite Betätigungsachse eine Rotationsrichtung (82) bereitstellt, deren Achse parallel zur Translationsrichtung (80) ist, und wobei die dritte Betätigungsachse und die vierte Betätigungsachse jeweils eine Schubrichtung (84, 86), zur Erfassung einer seitlichen Auslenkung senkrecht zur Translationsrichtung, oder eine Schwenkrichtung (98, 100), zur Erfassung einer seitlichen Neigung um zur Translationsrichtung senkrecht orientierte Achsen, bereitstellen.

8. Beobachtungsvorrichtung (10) nach einem der vorhergehenden Ansprüche, sofern direkt oder indirekt auf Anspruch 2 rückbezogen, wobei die Aufnahmepixelmenge (nₐ) ein ganzzahliges Vielfaches der Wiedergabepixelmenge (n_{b}) beträgt, und wobei die Aufnahmepixelmenge (nₐ) vorzugsweise ein Vierfaches, weiter bevorzugt ein Achtfaches der Wiedergabepixelmenge (n_{b}) beträgt, und wobei die Bildaufnahmeeinheit (18) vorzugsweise dazu ausgebildet ist, Bilddaten einer Rohdatenpixelmenge (nᵣ) zu erfassen, die größer als die Aufnahmepixelmenge (nₐ) ist, wobei die Rohdatenpixelmenge (nᵣ) einem Gesamterfassungsbereich (130) des Bildaufnehmers (26) entspricht, und wobei die Aufnahmepixelmenge (nₐ) als Ausschnitt der Rohdatenpixelmenge (nᵣ) gewählt ist.

9. Beobachtungsvorrichtung (10) nach einem der vorhergehenden Ansprüche, wobei die Bildverarbeitungseinheit (20) dazu ausgebildet ist, der Wiedergabeeinheit (16) eine interpolationsfrei darstellbare Wiedergabepixelmenge (n_{b}) bereitzustellen.

10. Beobachtungsvorrichtung (10) nach einem der vorhergehenden Ansprüche, wobei die Bedieneinheit (22) dazu ausgestaltet, mit einer sterilen Hülle überzogen zu werden, und wobei die Bedieneinheit (22), insbesondere das Eingabemodul (50), ferner dazu ausgestaltet ist, durch die sterile Hülle (240) hindurch bedient zu werden.

11. Beobachtungsvorrichtung (10) nach einem der vorhergehenden Ansprüche, wobei die Bedieneinheit (22) dazu ausgebildet ist, am Eingabemodul (50) eine haptische Rückmeldung bereitzustellen, insbesondere beim Erreichen von Grenzwerten oder Extremwerten von Parameterbereichen, die über das Eingabemodul (50) steuerbar sind.

12. Beobachtungsvorrichtung (10) nach einem der vorhergehenden Ansprüche, sofern direkt oder indirekt auf Anspruch 2 rückbezogen, wobei die Bildverarbeitungseinheit (20) dazu ausgebildet ist, Übersichtsbilder (62) zu erzeugen, die einen Bereich repräsentieren, der im Wesentlichen einem durch die Aufnahmepixelmenge (nₐ) umfassten Bereich (122) entspricht, wobei die Übersichtsbilder (62) eine Übersichtspixelmenge aufweisen, die kleiner als die Wiedergabepixelmenge (n_{b}) gewählt ist, und wobei die Übersichtsbilder (62) durch die Wiedergabeeinheit (16) zumindest zeitweise parallel zu den Wiedergabebildern (60) darstellbar sind, wobei die Übersichtsbilder (62) die Wiedergabebilder (60) abschnittsweise überdecken.

13. Beobachtungsvorrichtung (10) nach Anspruch 12, wobei die Wiedergabeeinheit (16) dazu ausgebildet ist, in den dargestellten Übersichtsbildern (62) einen Ausschnittsbereich (172) hervorzuheben, der einen durch die Wiedergabepixelmenge (n_{b}) umfassten Bereich (122) innerhalb der Aufnahmepixelmenge (nₐ) hervorhebt, und wobei sich der Ausschnittsbereich (172) in den Übersichtsbildern (62) bewegt, wenn durch Betätigung der Bedieneinheit (22) der durch die Wiedergabepixelmenge (n_{b}) abgedeckte Bereich (122) im durch die Aufnahmepixelmenge (nₐ) abgedeckten Bereich (112) bewegt wird.

14. Verwendung eines als Einhand-Eingabemodul ausgestalten Mehrachs-Eingabemodules (50) bei einer Bedieneinheit (22) einer medizinischen Beobachtungsvorrichtung (10) in Form eines Exoskopes (12) zur Steuerung von Bildaufnahmeparametern und Wiedergabeparametern und zur Steuerung eines Positionierantriebs (190, 192) zur Positionierung der Bildaufnahmeeinheit (18), wobei das Eingabemodul (50) zumindest in einem ersten Betriebsmodus und einem zweiten Betriebsmodus betreibbar ist, wobei im ersten Betriebsmodus eine direkte Steuerung von Bildaufnahmeparametern und Wiedergabeparametern ermöglicht ist, wobei das Eingabemodul (50) im zweiten Betriebsmodus steuerbar ist, um den Positionierantrieb zu steuern, und wobei das Eingabemodul (50) ein durch einen Bediener manipulierbares Betätigungselement (52, 90, 104) aufweist, das mehrere Bewegungsfreiheitsgrade (80, 82, 84, 86; 80, 82, 98, 100) für Eingaben bereitstellt, **dadurch gekennzeichnet, dass** verschiedene Achsen des Positionierantriebs (190, 192) gleichzeitig durch das Eingabemodul (50) steuerbar sind, und dass die direkte Steuerung von Wiedergabeparametern eine Positionierung eines Wiedergabebildes (60) in einem bereitgestellten globalen Aufnahmebild (58) umfasst.

## Claims

1. Observation device (10), in particular a medical observation device, comprising:
- an image capturing unit (18) with at least one image sensor (26), preferably a stereo image capturing unit with two image sensors,
- a display unit (16) configured to display image data provided by the image capturing unit (18),
- an image processing unit (20) for image processing tasks,
- a control unit (22) with a multi-axis input module (50), and
- a positioning drive (190, 192) for the image capturing unit (18),
wherein image capturing parameters and display parameters are controllable via the input module (50),
wherein the input module (50) is operable in at least a first operating mode and a second operating mode,
wherein in the first operating mode, direct control of image capturing parameters and display parameters is enabled,
wherein the input module (50) is actuable in the second operating mode to control the positioning drive (190, 192) to position the image capturing unit (18), and
wherein the input module (50) is configured as a single-hand input module and includes an actuating element (52, 90, 104) that is manipulable by an operator and that provides multiple degrees of freedom (80, 82, 84, 86; 80, 82, 98, 100) for inputs,
**characterized in that**
different axes of the positioning drive (190, 192) are controllable simultaneously by the input module (50), and
the direct control of display parameters includes positioning a display image (60) in a provided global capture image (58).

2. Observation device (10) according to claim 1, wherein the image capturing unit (18) is configured to provide capture images (58) of a predefined capture pixel quantity (nₐ), wherein the display unit (16) is configured to display display images (60) of a predefined display pixel quantity (n_{b}), wherein the capture pixel quantity (nₐ) is greater than or equal to the display pixel quantity (n_{b}), wherein image pixels (120) of the display pixel quantity (n_{b}) are obtainable from the capture pixel quantity (nₐ), wherein subsets of the capture pixel quantity (nₐ) are selectable to form the display pixel quantity (n_{b}) to provide views with different magnifications, and wherein the input module (50) is arranged to be coupled to the image capturing unit (18) to control at least one image capturing parameter.

3. Observation device (10) according to claim 2, wherein the input module (50) is coupled with the image capturing unit (18) and the image processing unit (20) to vary image capturing parameters of the image capturing unit (18) as well as to vary display parameters of the image processing unit (20), and wherein in the first operating mode, direct control of image capturing parameters and display parameters with the input module (50) is enabled, wherein in the second operating mode, control of additional peripheral functions with the input module (50) is enabled.

4. Observation device (10) according to any of the preceding claims, wherein the actuating element (52, 90, 104) is configured like a puck or knob, wherein the actuating element (52, 90, 104) is coupled with sensors (96) to detect a pull/push movement along a longitudinal axis (Z) of the actuating element (52, 90, 104), a rotational movement around the longitudinal axis (Z), and shift movements in a plane (X, Y) perpendicular to the longitudinal axis (Z) or swivel movements around swivel axes (X, Y) perpendicular to the longitudinal axis (Z).

5. Observation device (10) according to any of the preceding claims, wherein the actuating element (52, 90, 104) is coupled with at least one sensor (96) configured as a displacement sensor or force sensor, preferably with a plurality of sensors for multi-axial detection of deformations or movements.

6. Observation device (10) according to any of the preceding claims, wherein the actuating element (52, 90, 104) is configured as a four-axis actuating element, wherein an actuation of the first actuation axis (80) defines a magnification and a size of a section (122) of the display images (60) in the capture images (58) that is associated with the magnification, wherein an actuation of the second actuation axis (82) defines a focus adjustment, wherein an actuation of the third actuation axis (84) causes a movement of the area covered by the display images (60) within the area covered by the capture images (58) in a first direction (X), and wherein an actuation of the fourth actuation axis (86) causes a movement of the area covered by the display images (60) within the area covered by the capture images (58) in a second direction (Y) inclined to the first direction (X).

7. Observation device (10) according to claim 6, wherein the first actuation axis provides a translation direction (80), wherein the second actuation axis provides a rotation direction (82) whose axis is parallel to the translation direction (80), and wherein the third and fourth actuation axes each provide a shift direction (84, 86) for detecting a lateral displacement perpendicular to the translation direction or a swivel direction (98, 100) for detecting a lateral inclination about axes perpendicular to the translation direction.

8. Observation device (10) according to any of the preceding claims, provided they directly or indirectly refer to claim 2, wherein the capture pixel quantity (nₐ) is an integer multiple of the display pixel quantity (n_{b}), and wherein the capture pixel quantity (nₐ) preferably is four times, more preferably eight times, the display pixel quantity (n_{b}), and wherein the image capturing unit (18) is preferably configured to capture image data of a raw data pixel quantity (nᵣ) larger than the capture pixel quantity (nₐ), wherein the raw data pixel quantity (nᵣ) corresponds to a total capture area (130) of the image sensor (26), and wherein the capture pixel quantity (nₐ) is chosen as a section of the raw data pixel quantity (nᵣ).

9. Observation device (10) according to any of the preceding claims, wherein the image processing unit (20) is configured to provide the display unit (16) with a display pixel quantity (n_{b}) that is displayable interpolation-free.

10. Observation device (10) according to any of the preceding claims, wherein the control unit (22) is configured to be covered with a sterile cover, and wherein the control unit (22), in particular the input module (50), is further configured to be operated through the sterile cover (240).

11. Observation device (10) according to any of the preceding claims, wherein the control unit (22) is configured to provide haptic feedback on the input module (50), particularly when reaching limit values or extreme values of parameter ranges that are controllable via the input module (50).

12. Observation device (10) according to any of the preceding claims, provided they directly or indirectly refer to claim 2, wherein the image processing unit (20) is configured to generate overview images (62) representing an area corresponding essentially to an area covered by the capture pixel quantity (nₐ), wherein the overview images (62) have an overview pixel quantity smaller than the display pixel quantity (n_{b}), and wherein the overview images (62) are displayable at least temporarily parallel to the display images (60) by the display unit (16), wherein the overview images (62) partially cover the display images (60).

13. Observation device (10) according to claim 12, wherein the display unit (16) is configured to highlight a section area (172) within the displayed overview images (62) that corresponds to an area covered by the display pixel quantity (n_{b}) within the capture pixel quantity (nₐ), and wherein the section area (172) in the overview images (62) moves when the area covered by the display pixel quantity (n_{b}) within the area covered by the capture pixel quantity (nₐ) is moved by actuating the control unit (22).

14. Use of a multi-axis input module (50) configured as a single-hand input module in a control unit (22) of a medical observation device (10) in the form of an exoscope (12) to control image capturing parameters and display parameters and to control a positioning drive (190, 192) for positioning the image capturing unit (18), wherein the input module (50) is operable in at least a first operating mode and a second operating mode, wherein in the first operating mode, direct control of image capturing parameters and display parameters is enabled, wherein the input module (50) is controllable in the second operating mode to control the positioning drive, and wherein the input module (50) includes an actuating element (52, 90, 104) that is manipulable by an operator, providing multiple degrees of freedom (80, 82, 84, 86; 80, 82, 98, 100) for inputs, **characterized in that** different axes of the positioning drive (190, 192) are controllable simultaneously by the input module (50) and **in that** the direct control of display parameters includes positioning a display image (60) in a provided global capture image (58).

## Revendications

1. Dispositif d'observation (10), notamment dispositif d'observation médicale, qui comprend ce qui suit :
- une unité de capture d'images (18) comprenant au moins un capteur d'image (26), de préférence une unité de capture d'images stéréoscopique comprenant deux capteurs d'image,
- une unité de reproduction (16), qui est configurée pour reproduire des données d'image fournies par l'unité de capture d'images (18),
- une unité de traitement d'images (20) pour des processus de traitement d'images,
- une unité de commande (22) comprenant un module d'entrée (50) multi-axes, et
- un mécanisme d'entraînement de positionnement (190, 192) pour l'unité de capture d'images (18),
les paramètres de capture d'images et les paramètres de reproduction pouvant être commandés par le biais du module d'entrée (50),
le module d'entrée (50) pouvant fonctionner au moins dans un premier mode de fonctionnement et dans un deuxième mode de fonctionnement,
dans le premier mode de fonctionnement, une commande directe des paramètres de capture d'images et des paramètres de reproduction étant rendue possible,
le module d'entrée (50) pouvant être actionné dans le deuxième mode de fonctionnement pour commander le mécanisme d'entraînement de positionnement (190, 192) en vue de positionner l'unité de capture d'images (18), et
le module d'entrée (50) étant réalisé sous la forme d'un module d'entrée à une main et possédant un élément d'actionnement (52, 90, 104) manipulable par un opérateur, qui met à disposition plusieurs degrés de liberté de mouvement (80, 82, 84, 86 ; 80, 82, 98, 100) pour des entrées,
**caractérisé en ce**
**que** différents axes du mécanisme d'entraînement de positionnement (190, 192) peuvent être commandés simultanément par le module d'entrée (50), et
en ce que la commande directe de paramètres de reproduction comprend un positionnement d'une image de reproduction (60) dans une image de capture globale (58) mise à disposition.

2. Dispositif d'observation (10) selon la revendication 1, l'unité de capture d'images (18) étant configurée pour fournir des images capturées (58) ayant une quantité prédéfinie de pixels de capture (nₐ), l'unité de reproduction (16) étant configurée pour présenter des images de reproduction (60) d'une quantité prédéfinie de pixels de reproduction (n_{b}), la quantité de pixels de capture (nₐ) étant égale ou supérieure à la quantité de pixels de reproduction (n_{b}), des pixels d'image (120) de la quantité de pixels de reproduction (n_{b}) pouvant être prélevés de la quantité de pixels de capture (nₐ), des quantités partielles de la quantité de pixels de capture (nₐ) pouvant, en vue de fournir des vues ayant différents grossissements, être sélectionnées pour former la quantité de pixels de reproduction (n_{b}), et le module d'entrée (50) pouvant être couplé à l'unité de capture d'images (18) afin de commander au moins un paramètre de capture d'images.

3. Dispositif d'observation (10) selon la revendication 2, le module d'entrée (50) étant couplé à l'unité de capture d'images (18) et à l'unité de traitement d'images (20) pour agir sur l'unité de capture d'images (18) afin de faire varier les paramètres de capture d'images et sur l'unité de traitement d'images (20) afin de faire varier les paramètres de reproduction et, dans le premier mode de fonctionnement, une commande directe de paramètres de capture d'images et des paramètres de reproduction étant rendue possible avec le module d'entrée (50), une commande de fonctions périphériques supplémentaires étant rendue possible dans le deuxième mode de fonctionnement avec le module d'entrée (50).

4. Dispositif d'observation (10) selon l'une des revendications précédentes, l'élément d'actionnement (52, 90, 104) étant réalisé en forme de palet ou de pommeau, l'élément d'actionnement (52, 90, 104) étant couplé à des capteurs (96) afin de détecter un mouvement de traction/pression le long d'un axe longitudinal (Z) de l'élément d'actionnement (52, 90, 104), un mouvement de rotation autour de l'axe longitudinal (Z) et des mouvements de poussée dans un plan (X, Y) qui est orienté perpendiculairement à l'axe longitudinal (Z) ou des mouvements de pivotement autour d'axes de pivotement (X, Y) qui sont orientés perpendiculairement à l'axe longitudinal (Z).

5. Dispositif d'observation (10) selon l'une des revendications précédentes, l'élément d'actionnement (52, 90, 104) étant couplé à au moins un capteur (96), qui est réalisé sous la forme d'un capteur de déplacement ou d'un capteur de force, de préférence à une pluralité de capteurs pour la détection multiaxiale de déformations ou de mouvements.

6. Dispositif d'observation (10) selon l'une des revendications précédentes, l'élément d'actionnement (52, 90, 104) étant réalisé sous la forme d'un élément d'actionnement à quatre axes, un actionnement du premier axe d'actionnement (80) définissant un grossissement et une taille associée au grossissement d'une zone (122) des images de reproduction (60) dans les images capturées (58), un actionnement du deuxième axe d'actionnement (82) définissant un réglage de la mise au point, un actionnement du troisième axe d'actionnement (84) provoquant un déplacement de la zone (122) couverte par les images de reproduction (60) dans une zone (112) couverte par les images capturées (58) dans une première direction de déplacement (X), et un actionnement du quatrième axe d'actionnement (86) provoquant un déplacement de la zone (122) couverte par les images de reproduction (60) dans la zone (112) couverte par les images capturées (58) dans une deuxième direction de déplacement (Y) qui est inclinée par rapport à la première direction de déplacement (X).

7. Dispositif d'observation (10) selon la revendication 6, le premier axe d'actionnement fournissant une direction de translation (80), le deuxième axe d'actionnement fournissant une direction de rotation (82) dont l'axe est parallèle à la direction de translation (80), et le troisième axe d'actionnement et le quatrième axe d'actionnement fournissant respectivement une direction de poussée (84, 86), pour détecter une déviation latérale perpendiculaire à la direction de translation, ou une direction de pivotement (98, 100), pour détecter une inclinaison latérale autour d'axes orientés perpendiculairement à la direction de translation.

8. Dispositif d'observation (10) selon l'une des revendications précédentes, à la condition d'une référence directe ou indirecte à la revendication 2, la quantité de pixels de capture (nₐ) étant un multiple entier de la quantité de pixels de reproduction (n_{b}), et la quantité de pixels de capture (nₐ) étant de préférence un quadruple, encore de préférence un octuple de la quantité de pixels de reproduction (n_{b}), et l'unité de capture d'images (18) étant de préférence configurée pour acquérir des données d'image d'une quantité de pixels de données brutes (nᵣ) qui est supérieure à la quantité de pixels de capture (nₐ), la quantité de pixels de données brutes (nᵣ) correspondant à une zone d'acquisition totale (130) du capteur d'image (26), et la quantité de pixels de capture (nₐ) étant choisie en tant que portion de la quantité de pixels de données brutes (nᵣ).

9. Dispositif d'observation (10) selon l'une des revendications précédentes, l'unité de traitement d'images (20) étant configurée pour fournir à l'unité de reproduction (16) une quantité de pixels de reproduction (n_{b}) qui peut être représentée sans interpolation.

10. Dispositif d'observation (10) selon l'une des revendications précédentes, l'unité de commande (22) étant configurée pour être recouverte d'une enveloppe stérile, et l'unité de commande (22), notamment le module d'entrée (50), étant en outre configurée pour être commandée à travers l'enveloppe stérile (240).

11. Dispositif d'observation (10) selon l'une des revendications précédentes, l'unité de commande (22) étant configurée pour fournir un retour haptique au module d'entrée (50), notamment lorsque des valeurs limites ou des valeurs extrêmes de plages de paramètres, qui peuvent être commandées par le biais du module d'entrée (50), sont atteintes.

12. Dispositif d'observation (10) selon l'une des revendications précédentes, à la condition d'une référence directe ou indirecte à la revendication 2, l'unité de traitement d'images (20) étant configurée pour générer des images de vue d'ensemble (62) qui représentent une zone qui correspond sensiblement à une zone (122) comprise par la quantité de pixels de capture (nₐ), les images de vue d'ensemble (62) présentant une quantité de pixels de vue d'ensemble qui est choisie plus petite que la quantité de pixels de reproduction (n_{b}), et les images de vue d'ensemble (62) pouvant être représentées par l'unité de reproduction (16) au moins temporairement en parallèle avec les images de reproduction (60), les images de vue d'ensemble (62) recouvrant par portions les images de reproduction (60).

13. Dispositif d'observation (10) selon la revendication 12, l'unité de reproduction (16) étant configurée pour mettre en valeur, dans les images de vue d'ensemble (62) représentées, une zone de découpe (172) qui met en valeur une zone (122) comprise par la quantité de pixels de reproduction (n_{b}) à l'intérieur de la quantité de pixels de capture (nₐ), et la zone de découpe (172) se déplaçant dans les images de vue d'ensemble (62) lorsque, par un actionnement de l'unité de commande (22), la zone (122) couverte par la quantité de pixels de reproduction (n_{b}) est déplacée dans la zone (112) couverte par la quantité de pixels de capture (nₐ).

14. Utilisation d'un module d'entrée multi-axes (50) réalisé sous la forme d'un module d'entrée à une main dans une unité de commande (22) d'un dispositif d'observation (10) médical sous la forme d'un exoscope (12) pour la commande de paramètres de capture d'images et de paramètres de reproduction et pour la commande d'un mécanisme d'entraînement de positionnement (190, 192) en vue du positionnement de l'unité de capture d'images (18), le module d'entrée (50) pouvant fonctionner au moins dans un premier mode de fonctionnement et dans un deuxième mode de fonctionnement, une commande directe des paramètres de capture d'image et des paramètres de reproduction étant rendu possible dans le premier mode de fonctionnement, le module d'entrée (50) pouvant être commandé dans le deuxième mode de fonctionnement afin de commander le mécanisme d'entraînement de positionnement, et le module d'entrée (50) possédant un élément d'actionnement (52, 90, 104) manipulable par un opérateur, qui possède plusieurs degrés de liberté de mouvement (80, 82, 84, 86 ; 80, 82, 98, 100) pour des entrées, **caractérisé en ce que** différents axes du mécanisme d'entraînement de positionnement (190, 192) peuvent être commandés simultanément par le module d'entrée (50), et **en ce que** la commande directe de paramètres de reproduction comprend un positionnement d'une image de reproduction (60) dans une image de capture globale (58) mise à disposition.
